# EUROPEAN PATENT APPLICATION

(11) **EP 4 506 010 A1**
(43) Date of publication of application: **12.02.2025**
(21) Application number: 23784403.0
(22) Date of filing: 05.04.2023
(51) Int. Cl.: A61K 31/7115, C12N 15/113, A61K 48/00, A61P 21/00

(54) **ANTAGONISTS OF HUMAN MICRO RNA MIR-100, MIR-20, MIR-222, MIR-181 AND MIR-92 AND USES OF SAME**

(30) Priority: 06.04.2022 ES 202230311; 25.04.2022 ES 202230373
(71) Applicant: Universitat de València, 46010 Valencia (ES)
(72) Inventor: CERRO HERREROS, Estefanía, 46010 Valencia (ES); LLAMUSÍ TROISI, Beatriz, 46010 Valencia (ES); MORENO CERVERA, Nerea, 46010 Valencia (ES); GONZÁLEZ MARTÍNEZ, Irene, 46010 Valencia (ES); OVERBY, Sarah Joann, 46010 Valencia (ES); ARTERO ALLEPUZ, Rubén Darío, 46010 Valencia (ES)
(74) Representative: Hoffmann Eitle
(86) International application number: PCT/ES2023/070222
(87) International publication number: WO 2023/194641

(57) **Abstract**

Disclosed are antagonists of human microRNA miR-100, miR-20, miR-222, miR-181, and miR-92 and uses of same. The invention provides inhibitors of human microRNA miR-100, miR-20a, miR-222, miR-181c, and miR-92a, which are repressors of the genes MBNL1 and/or MBNL2. The use of the inhibitors as a drug, in particular against myotonic dystrophy type 1, is also provided.

## Description

### TECHNICAL FIELD

The invention relates to the field of biomedicine. In particular, the invention relates to molecules that are antagonists of human microRNAs miR-100, miR-20, miR-222, miR-181, and miR-92, and to the use of same as a drug, preferably as a drug for the treatment of myotonic dystrophy type 1.

### BACKGROUND OF THE INVENTION

Muscular dystrophy (MD) represents a group of over 30 genetic diseases characterized by a progressive weakness and degeneration of the skeletal muscles that control movement. Some forms of MD present in infancy or childhood, while others may not appear until middle age or later. The prognosis for people with MD varies depending on the type and progression of the disorder. Some cases may be mild and progress very slowly over a normal lifetime, while others result in severe muscle weakness, functional disability, and loss of the ability to walk. Within muscular dystrophies, a distinction is made between those in which patients have problems relaxing the muscles after a voluntary contraction, known as myotonia. There are two types of myotonic dystrophy, type 1 (DM1) and type 2 (DM2).

DM1 is further classified into three subtypes that sometimes have signs and symptoms in common: mild, classic, and congenital (present at birth). Symptoms of the mild form are the least severe, with a normal life expectancy. The classic form is characterized by muscle weakness and atrophy, inability of the muscles to relax quickly after contracting (myotonia), cataracts, and abnormal heart function. Some adults with the classic form may have a physical disability. Finally, the congenital form is characterized by severe generalized weakness at birth (hypotonia), which results in problems with breathing and can lead to premature death.

DM1 is caused by mutations in the *DMPK* gene and is inherited in an autosomal dominant manner. In particular, these mutations correspond to the expansion of a CTG trinucleotide repeat in the 3' non-coding region of the *DMPK* gene. A number of repeats greater than 34 CTG is considered abnormal, and molecular genetic testing detects pathogenic variants in almost 100% of affected individuals. Expression of expanded alleles in DM1 results in nuclear retention of mutant DMPK mRNA. The mutant transcripts sequester splicing factors that are similar to the *Drosophila* Muscleblind-like protein (MBNL), resulting in abnormal alternative splicing of a multitude of other transcripts and expression of fetal forms of the corresponding proteins in adults with DM1. The *Drosophila* Muscleblind-like protein has vertebrate homologs called MBNL1-3, which are also known for their ability to regulate splicing. MBNL1 is strongly expressed in skeletal and cardiac muscle tissue and during myoblast differentiation. Its expression is lower in other tissues such as brain, placenta, lungs, liver, kidney, and pancreas. MBNL2 has a largely overlapping expression and is detected in heart, brain, placenta, lungs, liver, skeletal muscle, kidneys, and pancreas. In turn, MBNL3 is expressed in placenta and satellite cells.

Although abnormalities in the splicing mechanism are considered the main factor underlying the pathogenesis of DM1, it has been shown that other mechanisms may also be involved in this disease, such as additional changes in gene expression, antisense transcripts, sequestration of other RNA-binding proteins or miRNAs, translation efficiency, alternative polyadenylation dysregulation, and miRNA dysregulation.

Currently there is no specific treatment for progressive weakness in people with DM1 and, therefore, it is of interest to explore new therapeutic strategies. Among the therapeutic approaches carried out in animal models of DM1, the most interesting results derive from blocking the interaction between MBNLs and toxic RNA using small molecules, peptides, morpholinos or antisense oligonucleotides, and gapmers to degrade mutant *DMPK* transcripts. However, a less explored alternative in DM1 is the therapeutic modulation of *MBNL1* and *MBNL2* gene expression, in particular the overexpression of said genes to increase the levels of said proteins, especially in tissues where they are normally transcribed. It is further preferred that said increased levels occur in at least one or more of the relevant tissues and organs where particularly significant symptoms of the disease appear, such as skeletal and smooth muscle, heart, and nervous system. In particular, the authors of the present invention have previously described that antagonists against human microRNAs miR-23-3p and miR-218-5p have therapeutic potential in the treatment of DM1 by being able to modulate endogenous MBNL proteins (patent ES2659845B1).

Therefore, given that microRNAs (commonly abbreviated as miRs) are known to be involved in numerous important biological processes such as metabolism, cell cycle, migration, epithelial-mesenchymal transition (EMT), cell differentiation, and survival, and pathological processes such as cancer, and since their regulation can be achieved with the use of antagonist molecules, the present invention describes antagonists of microRNAs miR-100, miR-20, miR-222, miR-181, and miR-92 to increase MBNL1 and MBNL2 protein levels, and proposes the use of such inhibitors in the treatment of DM1

### SUMMARY OF THE INVENTION

In one aspect, the present invention relates to an antagonist of human microRNA miR-100 capable of increasing the intracellular levels of MBNL1 and/or MBNL2 proteins, for use thereof in the treatment of myotonic dystrophy type 1. Preferably, the intracellular levels of MBNL1 and/or MBNL2 proteins are increased in a muscle cell, preferably in a muscle cell having a muscular dystrophy phenotype, preferably muscular dystrophy type 1. In a preferred embodiment, the antagonist is a molecule of an oligonucleotide nature and/or an oligonucleotide analog, preferably an antimiR, a blockmiR, or a microRNA sponge.

Preferably, the antagonist comprises a region of at least 7 contiguous nucleotide or nucleotide analog units which is at least 100% identical to the complementary sequence of SEQ ID NO: 1.

Preferably, the antagonist comprises a region of at least 15 contiguous nucleotide or nucleotide analog units which is at least 90% identical to the sequence complementary to any region present in SEQ ID NO: 1.

Preferably, the antagonist comprises a region of at least 7 contiguous nucleotide or nucleotide analog units which is 100% identical to the sequence complementary to the seed region of human microRNA miR-100 as defined in SEQ ID NO: 2.

Preferably, the antagonist comprises a first region of at least 7 contiguous nucleotide or nucleotide analog units which is 100% identical to the sequence complementary to the seed region of human microRNA miR-100 as defined in SEQ ID NO: 2, and wherein said antagonist further comprises a second region of at least 7 contiguous nucleotide or nucleotide analog units adjacent to the first region, wherein said second region is at least 90% identical to the sequence complementary to any region present in SEQ ID NO: 1.

Preferably, the antagonist comprises a region of at least 7 contiguous nucleotide or nucleotide analog units which is at least 95% identical to SEQ ID NO: 3. Preferably, the antagonist comprises a region of at least 15 contiguous nucleotide or nucleotide analog units which is 100% identical to any region present in SEQ ID NO: 3. Preferably, the antagonist consists of SEQ ID NO: 3.

Preferably, the antagonist:
i) comprises a region which is at least 95% identical to SEQ ID NO: 3,
ii) comprises at least one nucleotide in turn comprising one or more chemical modifications in the ribose moiety, in the phosphate bond, or in both, and
iii) optionally has at the 5' end and/or at the 3' end one or more additional molecules, wherein preferably said one or more additional molecules are not deoxyribonucleotide or ribonucleotide derivatives, and wherein preferably it is cholesterol.

Preferably, the antagonist comprises a region of at least 8 contiguous nucleotide or nucleotide analog units which is at least 95% identical to any region present in SEQ ID NO: 8, 13, or 26. Preferably, the antagonist comprises a region of at least 15 contiguous nucleotide or nucleotide analog units which is at least 95% identical to any region present in SEQ ID NO: 8, 13, or 26. Preferably, the antagonist consists of SEQ ID NO: 8, 13, or 26.

In another aspect, the present invention relates to a composition comprising at least one antagonist of human microRNA miR-100 as defined in the preceding aspect or any of the preferred embodiments thereof, for use thereof in the treatment of myotonic dystrophy type 1. Preferably, said composition is a pharmaceutical composition further comprising a pharmaceutically acceptable vehicle and/or one or more pharmaceutically acceptable excipients.

### BRIEF DESCRIPTION OF THE FIGURES

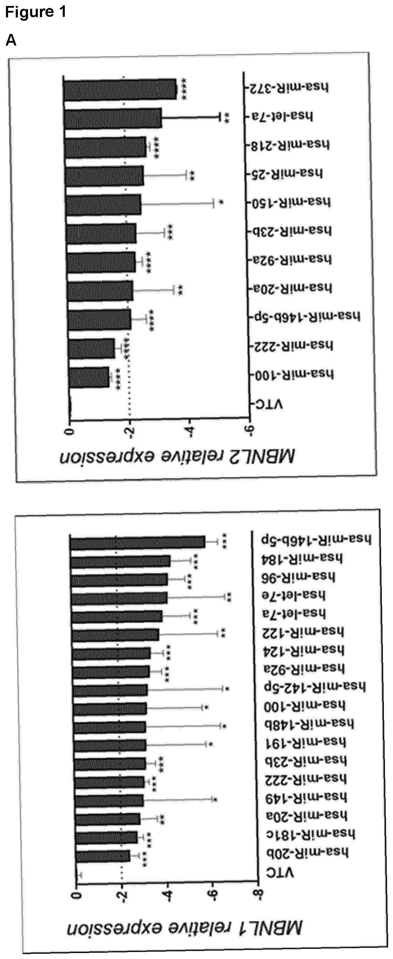

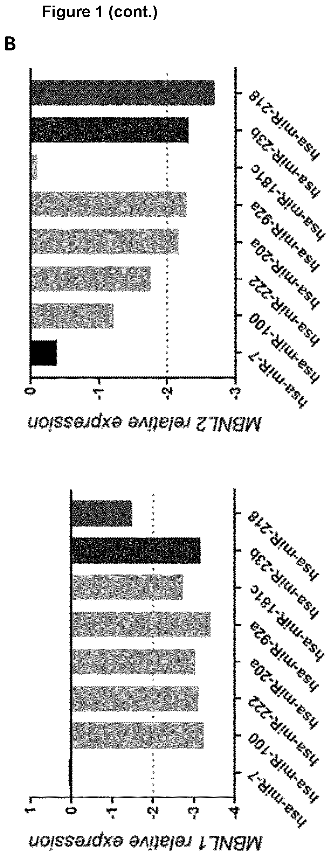

**Figure 1****: Expression levels of genes of interest in HeLa cells.** Base-2 logarithmic representation (log2) of the relative expression level of the of *MBNL1* (left) and *MBNL2* (right) transcripts in HeLa cells treated with (A) the mimetics from the initial screening performed with the SureFind Transcriptome PCR Array miRNA plates and (B) selection of those microRNAs of interest, as well as miR-23b and -218 as a comparison. For both genes, the reference value is that detected in HeLa cells treated without any mimetic and the endogenous gene used to normalize is GAPDH. *p<0.05 ,**p<0.01,***p<0.001 (Student's t-test).

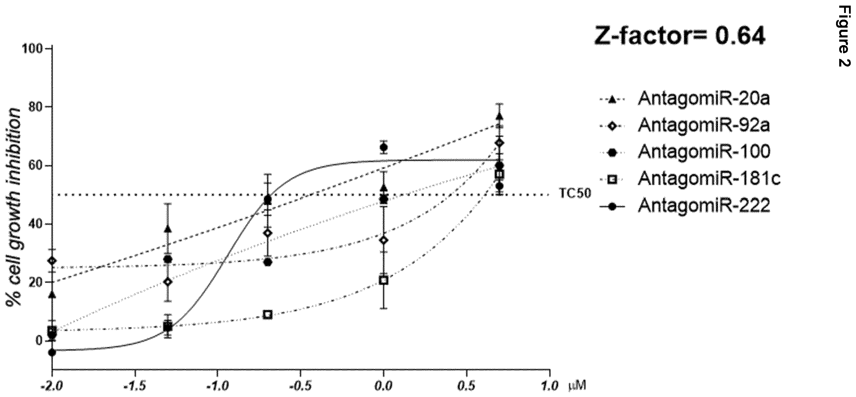

**Figure 2****: Toxicity measurements in the cell model of the disease.** Graphical representation of the percentage of cell viability inhibition at 72 h, obtained in patient cells caused by toxicity associated with a dose-response assay with increasing amounts of the antagomiRs miR100, miR-20a, miR-181c, miR-222, and miR-92a, with respect to the base-10 logarithm of the nanomolar concentration of the compound. The amounts tested in the cells are indicated at the bottom of the corresponding curves. The dotted line indicates the dose corresponding to TC50. Z-factor of the assay: 0.64. Symbols represent the mean ± SEM.

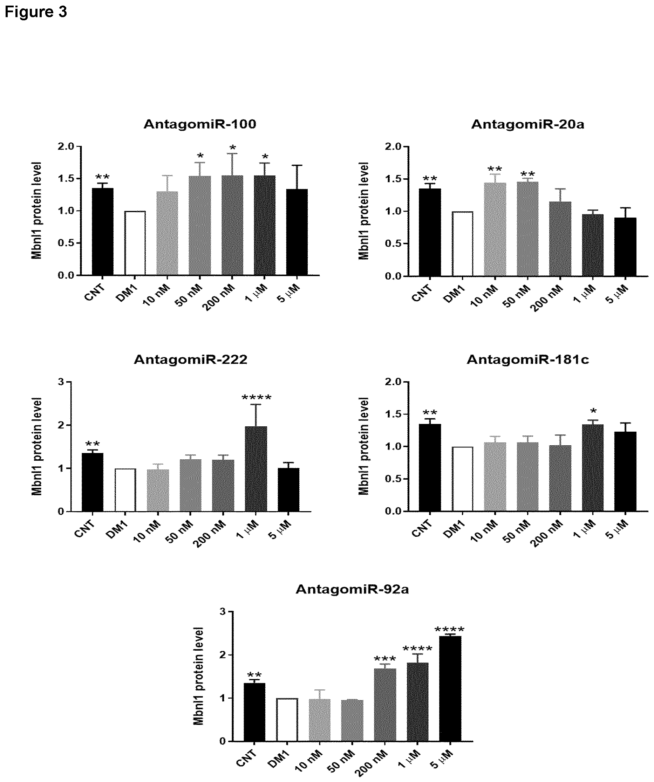

**Figure 3****: Levels of protein of interest in the cell model of the disease.** Relative quantification of the levels of MBNL1 protein, measured by means of Quantitative Dot Blot, in DM1 muscle cells transfected with the concentrations and antagomiRs indicated and compared with the levels detected in control muscle cells (CNT). Bars represent the mean ± SEM. GAPDH was used as the endogenous control to normalize expression. * p<0.05, ** p<0.01, *** p<0.001 (one-way ANOVA test)

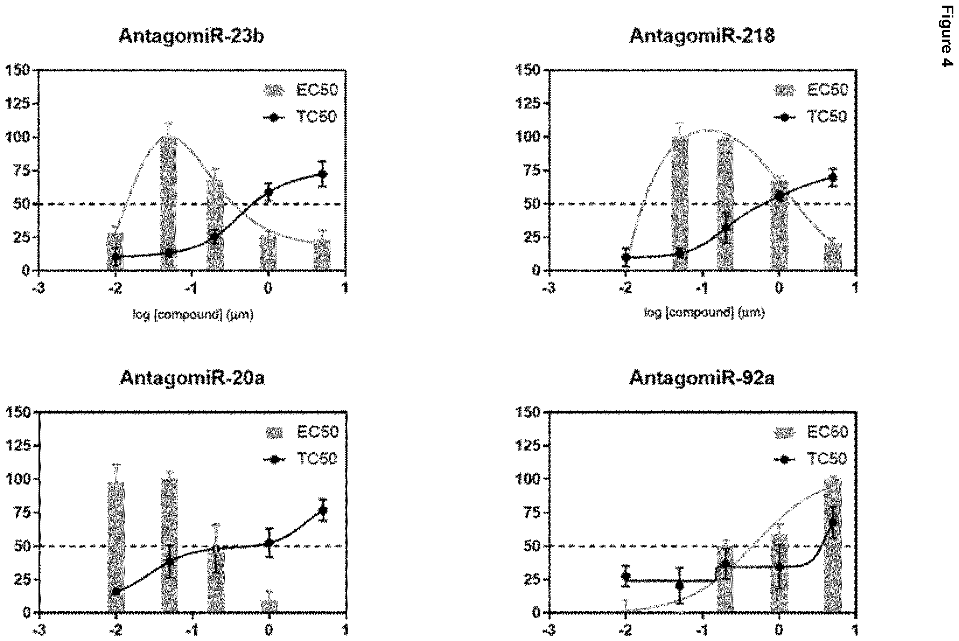

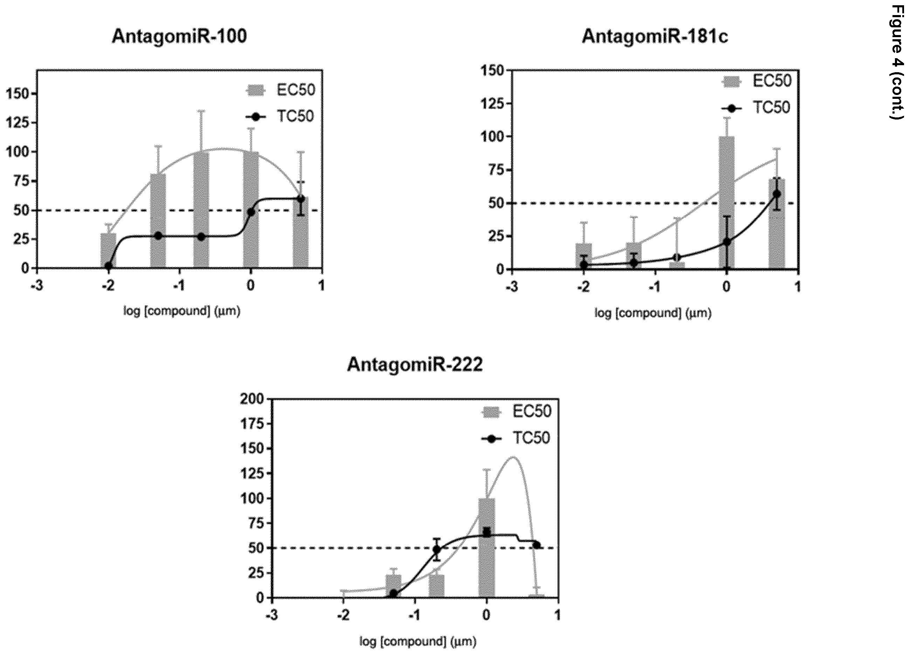

**Figure 4****. Evaluation of the activity and toxicity of the different antagomiRs in the cell model of the disease.** Representation of the toxicity (percentage of cell viability inhibition, black line) and the activity (percentage increase in the expression of MBNL1, gray line and bars) in DM1 cells after transfection with the different antagomiRs at the different concentrations tested (each in triplicate). Each symbol and bar represents the mean ± SEM

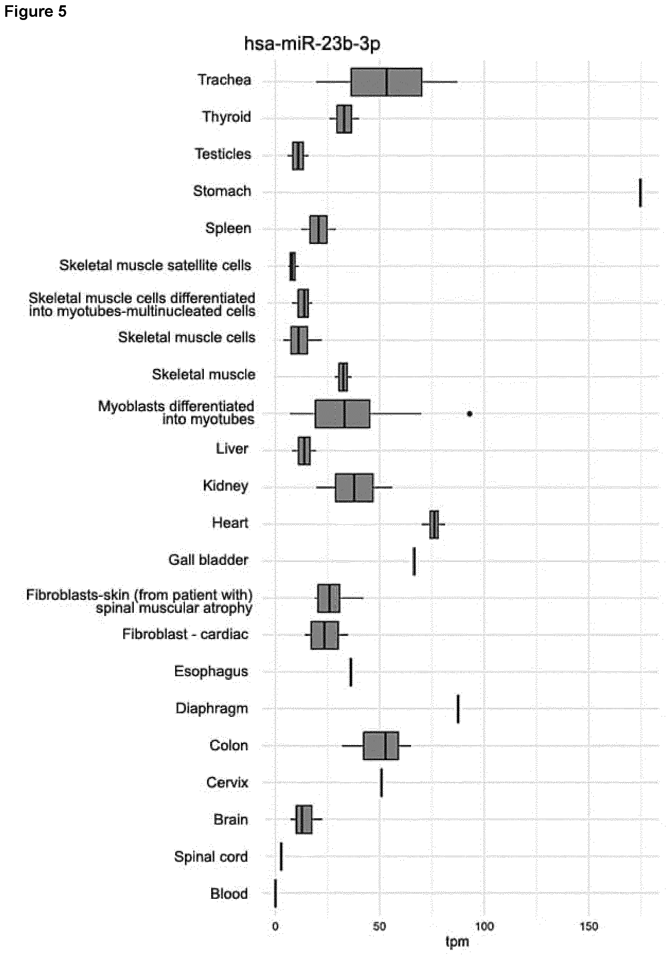

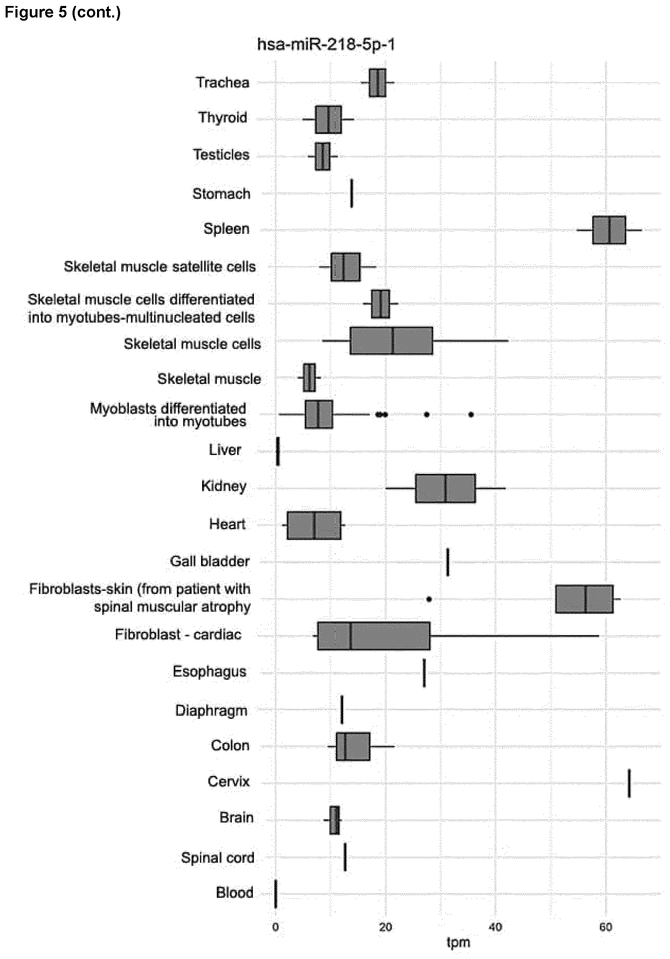

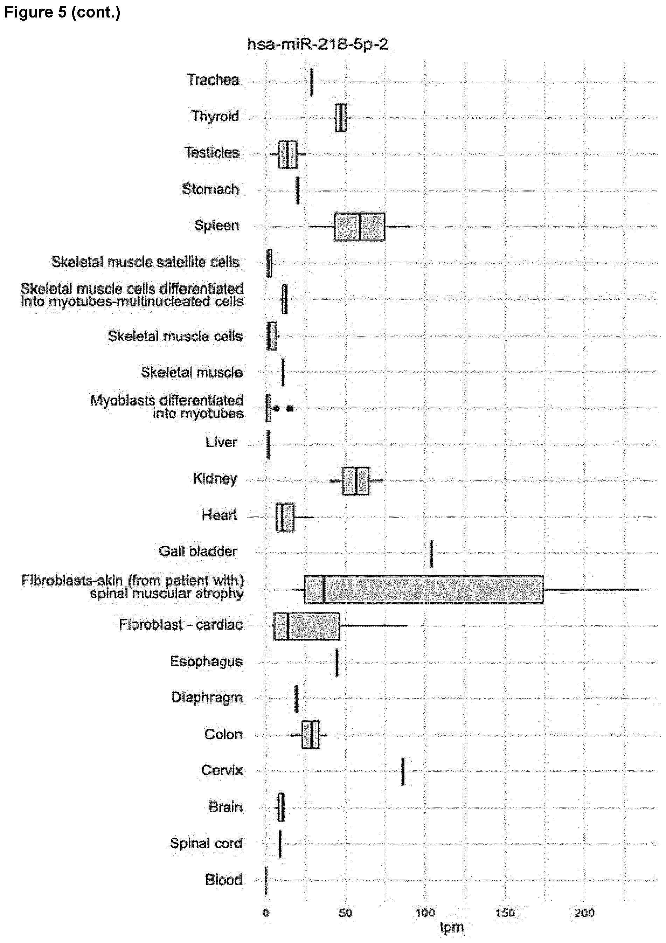

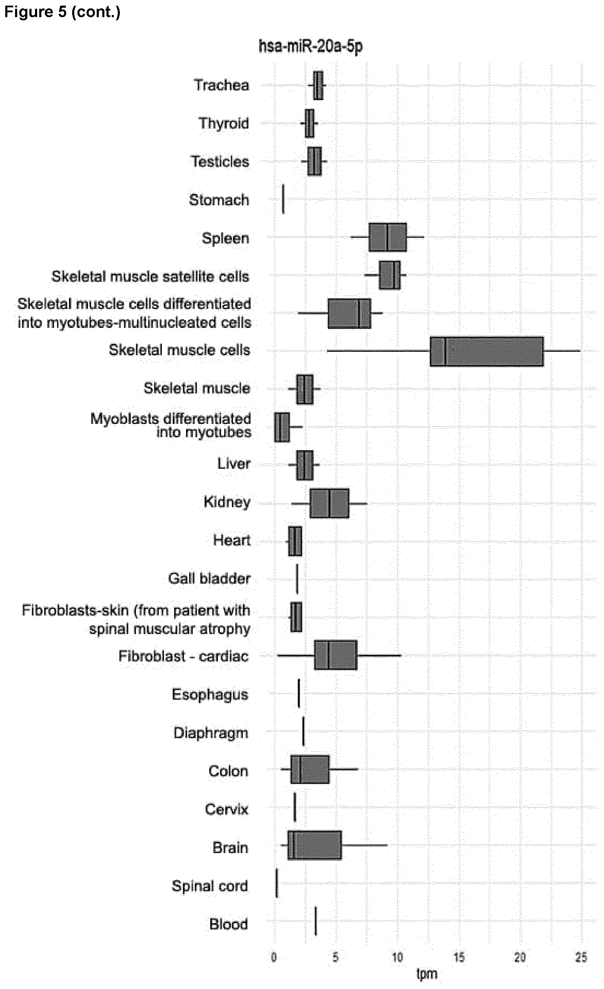

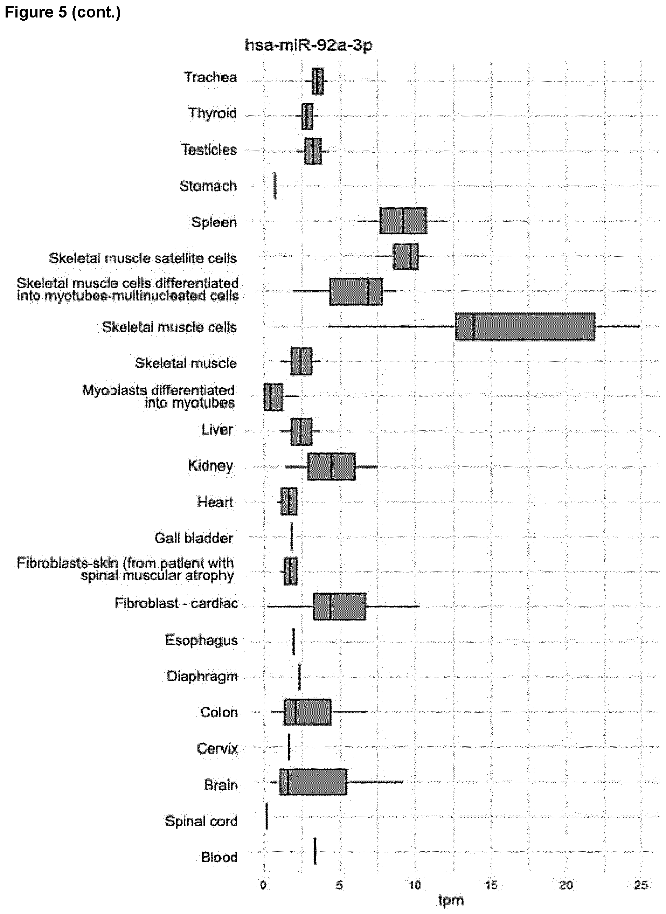

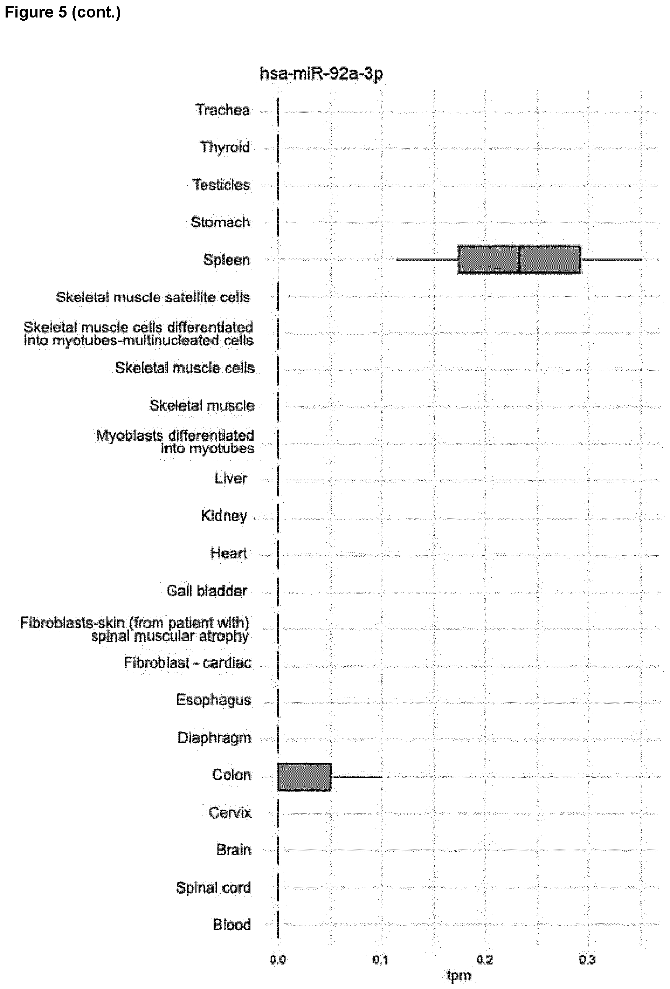

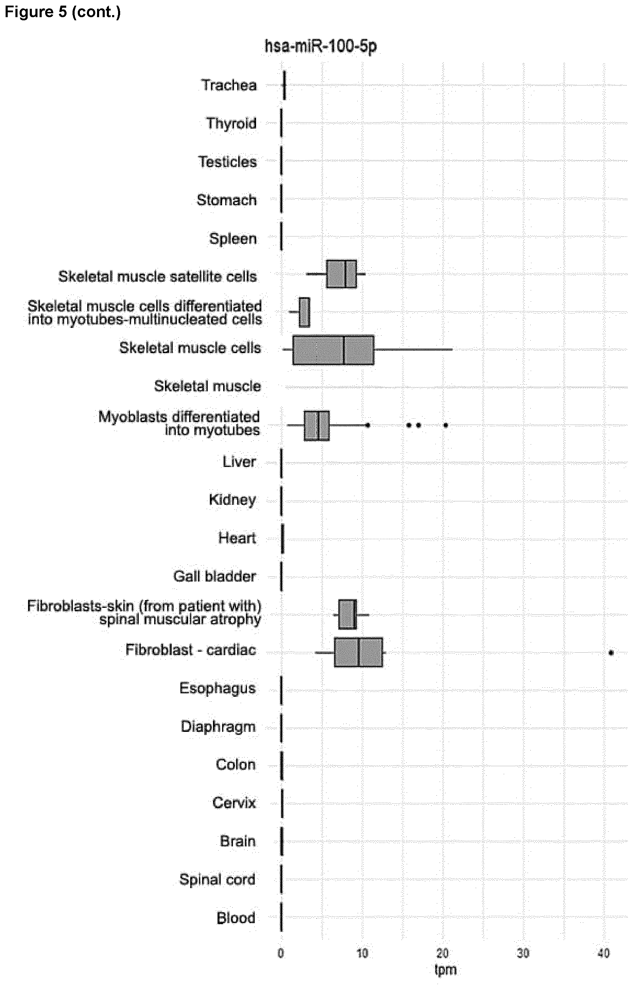

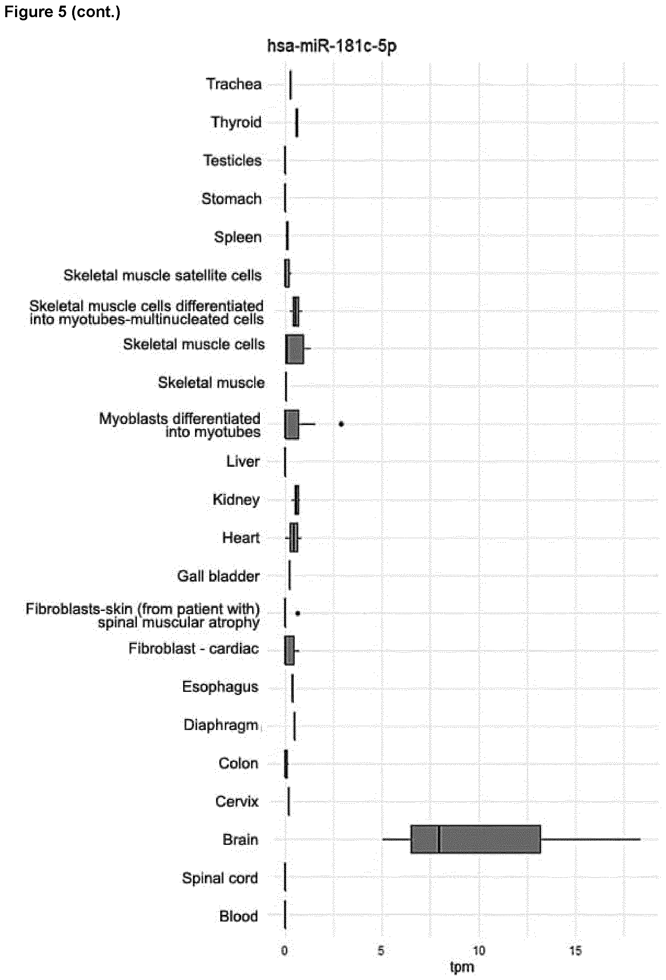

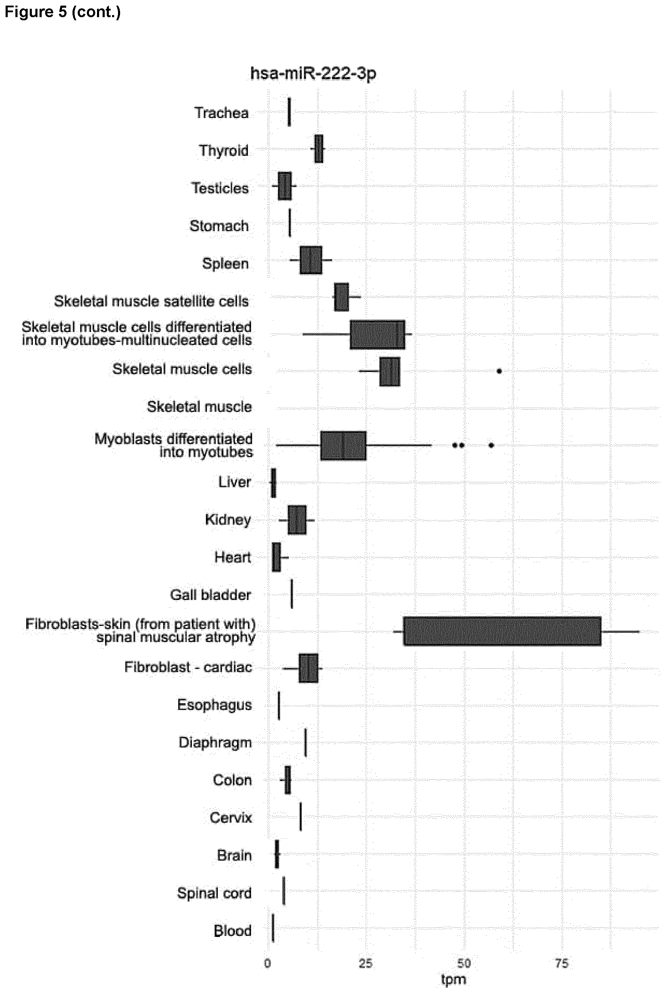

**Figure 5****: Expression of the microRNAs of interest in different tissues.** Expression levels of miR-23b-3p, miR-218-5p-1, miR-218-5p-2, miR-20a-5p, miR-92a-3p, miR-100-5p, miR-181c-5p, and miR-222-3p in transcripts per million (TPMs), in the different tissues of interest: trachea, thyroid, testicles, stomach, spleen, different types of muscle cells, skeletal muscle, liver, kidney, heart, bladder, esophagus, diaphragm, colon, cervix, brain, and blood. The data is from the sequencing of 100 samples.

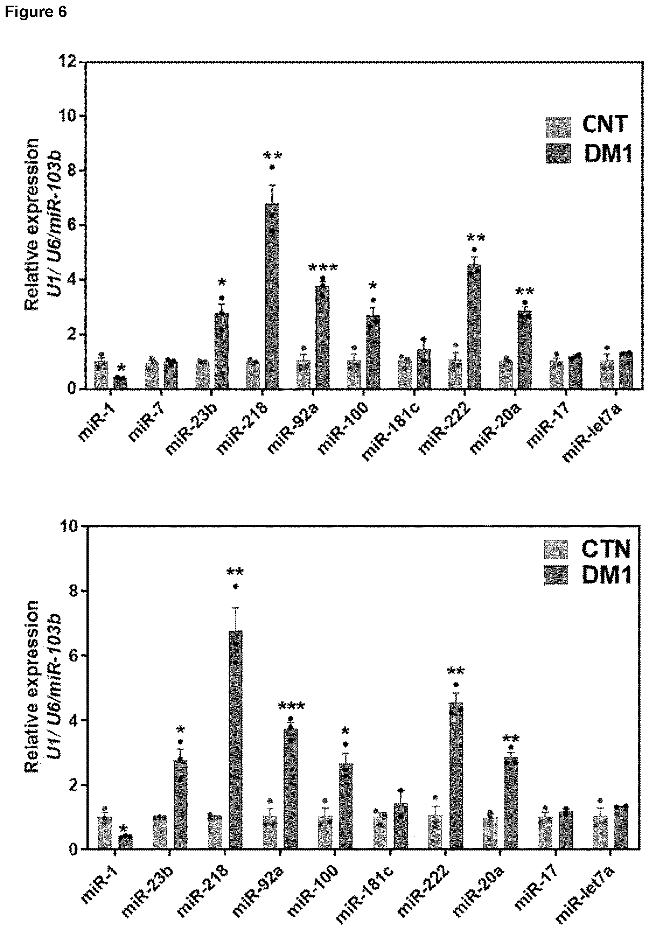

**Figure 6****: Demonstration of the overexpression of the different miRNAs in a cell model of the disease.** The expression levels of the miRNAs indicated in DM1 muscle cells and controls from Dr. Denis Furling (Arandel *et al.* 2017) are shown. These levels have been quantified by quantitative PCR and have been normalized to the expression of endogenous controls U1, U6, and miR-103. Bars represent the mean ± SEM. p<0.05 ,**p<0.01,***p<0.001 (Student's t-test).

### DEFINITIONS

It should be noted that, as used herein, the singular forms "a", "an", and "the" include plural references unless the context clearly indicates otherwise. Furthermore, unless otherwise indicated, the term "at least" preceding a series of elements should be understood to refer to each element in the series. Those skilled in the art will recognize, or will be able to determine using no more than routine experimentation, many equivalents to the specific embodiments of the invention described herein. It is intended that said equivalents be encompassed by the present invention.

As used herein, the combined term "and/or" among multiple listed elements is understood to encompass both individual and combined options. For example, when two elements are joined by "and/or", a first option refers to the applicability of the first element without the second element. A second option refers to the applicability of the second element without the first element. A third option refers to the applicability of the first and second elements together. It is understood that any of these options fall within the meaning and, therefore, satisfy the requirement of the term "and/or" as used herein. It is also understood that the simultaneous applicability of more than one of the options falls within the meaning and, therefore, satisfies the requirement of the term "and/or".

Throughout this specification and the claims that follow, unless the context requires otherwise, the word "comprises" will be understood to imply the inclusion of an integer or step or group of integers or steps, but not the exclusion of any other integer or step or group of integers or steps. As used herein, the term "comprising" may be replaced with the term "containing" or "including" or "having". The terms "comprising", "containing", "including", "having", whenever used herein in the context of an aspect or embodiment of the present invention, may be replaced with the term "consisting of", although this is less preferred. As used herein, "consisting of" excludes any element, step, or ingredient not specified in the claim element.

The term "hybridization" is used to refer to the structure formed by 2 or more independent RNA or DNA strands forming a double or triple stranded structure by means of pairing the bases of one strand to the other. These base pairs are considered G-C, A-U/T, and G-U/T. (A-Adenine, C-Cytosine, G-Guanine, U-Uracil, T-Thymine). As in the case of complementarity, hybridization can be full or partial.

The terms "complementary" and "complementarity" are interchangeable and refer to the ability of oligonucleotides to form base pairs with each other. Base pairs are normally formed by means of hydrogen bonds between nucleotide units in antiparallel polynucleotide strands or regions. Complementary polynucleotide strands or regions can form base pairs, for example, as in the way of Watson-Crick (e.g., A to T, A to U, C to G). A 100% (or perfect) complementarity refers to the situation in which each nucleotide unit of one oligonucleotide strand or region can form hydrogen bonds or bridges with each nucleotide unit of a second polynucleotide region or strand. Partial or non-perfect complementarity refers to the situation in which some, but not all, nucleotide units of two strands or two regions can form hydrogen bonds with each other and can be expressed as a percentage. The terms "sequence identity" or "percent identity" in the context of two or more nucleotides, polypeptides, or proteins refer to two or more sequences or subsequences that are the same ("identical") or have a specific percentage of nucleobases or amino acids that are identical ("percent identity") when compared and aligned to obtain maximum correspondence with a second molecule, as measured using a local sequence comparison algorithm (e.g., by means of BLAST alignment or any other local algorithm known to skilled persons), or alternatively, by visual inspection. It should be noted that percent identity as used here is measured in the context of a local alignment, i.e., it is based on the alignment of regions of local similarity between sequences, unlike a global alignment, the objective of which is to align two sequences over their entire length. Therefore, in the context of the present invention, percent identity is calculated solely on the basis of a local alignment comparison algorithm.

In the context of the present invention, complementarity is considered to occur under stringent hybridization conditions. "Stringent hybridization conditions" refers to conditions in which nucleotides that have homology with the target sequence preferably hybridize with the target sequence, and nucleotides that do not have homology with the target sequence do not substantially hybridize. The stringent conditions depend on the sequence, as longer sequences hybridize specifically at higher temperatures. Generally speaking, stringent conditions are chosen to be approximately 5°C lower than the thermal melting temperature (Tm) of the specific sequence. Tm is the temperature at which 50% of the nucleotides complementary to the target sequence hybridize with the target sequence in equilibrium at a prescribed ionic strength, pH, and nucleic acid concentration.

"Fragment" or "region" of a sequence is understood to mean a portion of a polypeptide or nucleic acid molecule preferably containing at least 10%, 20%, 30%, 40%, 50%, 50%, 60%, 70%, 80%, 90%, 95%, or more of the total length of the reference nucleic acid molecule or polypeptide. A fragment may contain at least 5, 6, 7, 8, 8, 9, 10, 10, 11, 11, 12, 13, 13, 14, 14, 15, 16, 17, 18, 19, or 20 nucleotides with respect to the reference sequence.

"MBNL1" is understood to mean the Muscleblind-like Splicing Regulator 1 protein encoded by the *MBNL1* gene. The term "MBNL1" as used in the present description includes all variants encoded by this gene.

"MBNL2" is understood to mean the Muscleblind-like Splicing Regulator 2 protein encoded by the *MBNL2* gene. The term "MBNL2" as used in the present description includes all variants encoded by this gene.

"Myotonic dystrophy" (DM) refers to a group of inherited disorders called muscular dystrophies. There are two main types of myotonic dystrophy: type 1 (DM1) and type 2 (DM2). Myotonic dystrophy type 1 (DM1), as defined herein, is a multisystem disorder that affects skeletal and smooth muscle, as well as other tissues, such as the eye, heart, endocrine system, and central nervous system. The clinical manifestations of DM1 span a continuum from mild to severe and have been broadly categorized into three phenotypes: mild, classic, and congenital. Mild DM1 is characterized by cataracts and mild myotonia (sustained muscle contraction); life expectancy is normal. Classic DM1 is characterized by muscle weakness and atrophy, myotonia, cataracts, and often cardiac conduction abnormalities; adults may become physically disabled and may have a shorter life span. Congenital DM1 is characterized by hypotonia and severe generalized weakness at birth, often with respiratory failure and premature death; intellectual disability is common.

"Treatment of myotonic dystrophy" refers to the treatment of symptomatic or asymptomatic myotonic dystrophy, at least partially restoring the phenotype of a patient with such a disease to a non-pathological state. "Prevention of myotonic dystrophy" involves measures taken to prevent the development of myotonic dystrophy (i.e., preventing the onset of histopathology and/or symptomatology-associated myotonic dystrophy).

An "effective dose" or "therapeutically effective dose" is an amount sufficient to achieve a beneficial or desired clinical outcome. In the context of the present invention, the following definitions of TC50, EC50, Emax, and Tlndex have been applied:
TC50 refers to the dose at which antagomiR produces toxic effects (lack of cell viability) in 50 percent of the cells on which it is being tested in a 72-hour period.

EC50 refers to the concentration of the antagomiR required to reach 50 percent of its maximum activity in a 72-hour period.

Emax refers to the maximum activity (in this case, expression of MBNL1 or MBNL2 proteins measured in fold change) of the antagomiR in a 72-hour period.

Tindex refers to a measure of a drug's safety margin and efficacy. It is expressed numerically as a ratio between the concentration of the antagomiR that causes cell death (TC50) taking into account its Emax and the dose that causes the desired therapeutic effect (EC50).

"Expression vector" means a nucleic acid molecule (usually a plasmid), a virus, an RNA molecule, a minichromosome, liposome, exosome, or a cell, designed to express or deliver a specific biomolecule, in the present case an antagonist. The expression vector is introduced into a host cell using well-known techniques such as infection or transfection, including calcium phosphate transfection, liposome-mediated transfection, electroporation, and sonoporation. The expression constructs and methods for their generation and use to express a desired protein are known to one skilled in the art.

### DETAILED DESCRIPTION OF THE INVENTION

It is widely accepted that myotonic dystrophy type 1 (DM1) is caused, among other molecular defects, by the sequestration and consequent lack of function of MBNL1 and 2 proteins, which are abnormally sequestered by CUG expansions in disease-causing mutant *DMPK* transcripts. In the present invention, the inventors set out to identify the miRNAs that endogenously repress the expression of MBNL1 and 2 in order to, by means of inhibitors thereof (antagomiRs and blockmiRs), find those molecules that could prevent such inhibition and thereby increase the levels of MBNL1 and 2 proteins, the function of which is reduced under pathological conditions. AntagomiR and antimiR are used synonymously in the present description.

For this purpose, a screening was performed in HeLa cells in which a set of 90 agomiRs (miRNA mimetics) were overexpressed and the levels of *MBNL1* and *MBNL2* transcripts were quantified by means of real-time PCR. A total of 5 miRNAs were found to be capable of repressing the expression of MBNL1 and/or MBNL2 under these conditions: hsa-miR-20a-5p (SEQ ID NO:4), hsa-miR-181c-5p (SEQ ID NO: 5), hsa-miR-222-3p (SEQ ID NO:6), hsa-miR-100-5p (SEQ ID NO:3), hsa-miR-92a-3p (SEQ ID NO: 7). Next, antisense oligonucleotides were designed against each of the identified miRNAs, known as antagomiRs, and tested in patient-derived muscle cells. The relative quantification of MBNL1 protein levels can be seen in Figure 3, wherein it is shown that while most cause only less notable increases, some antagomiRs are active at lower concentrations than others, for example, antagomiR-20a or - 100, which at 10 nM already recovers to normal levels (see control), and others are able to double expression in DM1 cells (antagomiR-92a).

Then, the inventors proceeded to evaluate toxicity in the cell model of the disease. Table 3 shows the TC50, EC50, Emax, and Tlndex values of the antagonists described in the present invention, wherein a very high Tlndex can be observed in the case of antagomiR-100, derived from its low toxicity (TC50) and EC50.

In view of these above results, and taking into account that other antagonists that raise MBNL1 levels and are used for the treatment of DM1 (ES2659845B1) are known in the prior art, it can be concluded that antagonists of miR-100-5p, miR-20a-5p, miR-222-3p, miR-181c-5p, and miR-92a-3p have significant therapeutic potential by increasing MBNL1 levels and can be used as drugs, particularly in the treatment of patients with DM1. Furthermore, results were also obtained on the ability of these antagonists to decrease the relative expression levels of MBNL2, as shown in Figure 1.

Therefore, a **first aspect** of the invention relates to an antagonist of a human microRNA, wherein said human microRNA is selected from the group of miR-100, miR-20a, miR-222, miR-181c, and miR-92a, and wherein said antagonist is capable of increasing the intracellular levels of the MBNL1 and/or MBNL2 proteins, and/or the intracellular levels of the transcripts (mRNA) of the genes encoding the MBNL1 and/or MBNL2 proteins. In a preferred embodiment, the antagonist of a human microRNA is an antagonist of human microRNA miR-100.

In another preferred embodiment, the antagonist of human microRNA miR-100 is capable of increasing the intracellular levels of the MBNL1 and/or MBNL2 proteins and/or the intracellular levels of the transcripts of the genes encoding the MBNL1 and/or MBNL2 proteins in a muscle cell, preferably in a muscle cell having a muscular dystrophy phenotype, preferably type 1. As used herein, the expression "capable of increasing the levels of the MBNL1 and/or MBNL2 proteins or their transcripts" refers to an increase, preferably a statistically significant increase, in the intracellular levels of said proteins (or any of their variants) or in the intracellular levels of the mRNA encoding them, caused by treatment with the antagonists of the present invention, wherein said increase is in comparison to the levels of a control or untreated cell or cell population. Control cell or cell population refers to cells not treated with the antagonists of the invention and having an abnormally reduced MBNL1 and/or MBNL2 function due to muscular dystrophy type 1 or due to the presence of at least one of the miRNAs acting negatively on their expression. In statistics, a result or effect is statistically significant when it is unlikely to have been due to chance. The significance level of a statistical test is a statistical concept associated with the verification of a hypothesis. Said test is defined as the probability of making the decision to reject the null hypothesis when it is true (a decision known as type I error, or false positive). The decision is often made using the p-value: if the p-value is below the significance level, then the null hypothesis is rejected. The lower the p-value, the more significant the result. Preferably, increased intracellular levels of proteins or their transcripts are considered to be statistically significant compared to the levels of said proteins or transcripts in control cell(s) when the p-significance levels are equal to or less than 0.05, 0.01, and 0.001.

Preferably, the control cells are muscle cells having a muscular dystrophy type 1 phenotype. Preferably, the intracellular levels of MBNL1 and/or MBNL2 proteins and/or transcripts thereof increase by at least 1.2, 1.3, 1.4, 1.5, 1.6, 1.7, 1.8, 1.9, 2, 3, 4, 5, 6, 7, 8, 9, 10, or more fold changes with respect to the levels of said proteins/transcripts present in a control or untreated cell or cells .

In that sense, the present inventors propose the modulation of endogenous MBNL proteins by causing the sequestration of at least one of the miRNAs that act negatively on their expression, thereby giving rise to an up-regulation and, as a consequence, to an increase in the intracellular levels of endogenous MBNL1 and/or MBNL2 proteins or their transcripts (mRNAs). Thus, the aim is to modulate the endogenous protein by means of silencing or decreasing the activity of human microRNAs miR-100, miR-20a, miR-222, miR-181c, or miR-92a.

As used herein, "antagonist" refers to any molecule capable of blocking the action of human microRNAs, preferably miR-100, miR-20a, miR-222, miR-18c1, and miR-92a, i.e., compounds, regardless of their nature, that are capable of producing a decrease in the endogenous activity of said miRNAs, and thereby increasing the intracellular levels of MBNL1 and/or MBNL2 proteins and/or their transcripts. Since the present invention focuses on decreasing the activity of human microRNAs miR-100, miR-20a, miR-222, miR-181c, and miR-92a, said repressor capacity will be diminished by the presence of the inhibitors, silencers, or blockers thereof, which are all referred to as antagonists. Therefore, similarly, the effect produced by an inhibitor, silencer, or blocker entails and signifies an antagonism of the action of the same. Preferably, the miR-100, miR-20a, miR-222, miR-181c, and miR-92a the action of which is blocked by the antagonist of the present invention are specifically miR-100-5p, miR-20a-5p, miR-222-3p, miR-181c-5p, and miR-92a-3p.

In one embodiment, the antagonist capable of blocking the action of human microRNA, preferably miR-100, miR-20a, miR-222, miR-18c1, and miR-92a, is a molecule, preferably a low molecular weight organic compound.

In a preferred embodiment, the antagonist is a molecule of an oligonucleotide nature and/or an oligonucleotide analog. As used in the present specification, the terms "molecule of an oligonucleotide nature" or "oligonucleotide" refers to molecules resulting from the binding of usually not more than 50 units of the monomers that give rise to the molecules known in abbreviated form as DNA or RNA, which are monomers or oligomers made up of a phosphate group, the nitrogenous bases adenine (A), cytosine (C), guanine (G), or uracil (U) or thymine (T), and the pentose known as ribose (in the case of oligoribonucleotides in the case of RNA) or deoxyribose (in the case of oligodeoxyribonucleotides, or DNA). Oligodeoxynucleotides (DNA) and oligoribonucleotides (RNA) are therefore considered to be included among oligonucleotides. By common usage, molecules the units of which include the nucleotide inosine and molecules that are a mixture of DNA/RNA because they contain regions of both types are also considered to be included within said definition.

In a preferred embodiment, the antagonist is a molecule of an oligoribonucleotide nature (a oligoribonucleotide) and/or an oligoribonucleotide analog. As used in the present specification, the terms "molecule of an oligoribonucleotide nature" or "oligoribonucleotide" refer to molecules resulting from the binding of usually not more than 50 units of the monomers that give rise to the molecule known in abbreviated form as RNA, which are monomers made up of a phosphate group, the nitrogenous bases adenine (A), cytosine (C), guanine (G), or uracil (U), and pentose. By common usage, molecules the units of which include the nucleotide inosine are also considered to be included within said definition.

As used in the present invention, the terms "oligonucleotide analog" and "oligoribonucleotide analog" encompass molecules derived from oligonucleotides and oligoribonucleotide, respectively, incorporating artificially introduced chemical modifications, such as those described in Antisense part III: chemistries Aug 28, 2018 ericminikel • Cambridge, MA. (https://www.cureffi.org/2018/08/28/antisense-part-iii-chemistries/). Preferably, the modifications are among one of the following modifications: i) a chemical modification in at least one of the component units, either in the phosphate group, the pentose, or one of the nitrogenous bases; or ii) replacement of one or more of the basic components constituting the oligonucleotide with a different chemical structure but with the same function. In the case of ii), these include, without limitation, the use of morpholino rings replacing pentoses to arrange the nitrogenous bases in their suitable spatial arrangement, or the replacement of phosphodiester internucleotide bonds with phosphorothioate internucleotide bonds. Furthermore, other modifications iii) also include those consisting of the addition of groups of a non-nucleotide nature at the 5' and/or 3' ends; and/or iv) the addition of further oligonucleotide sequences or oligonucleotide analogs (i.e., not complementary to the target microRNA) at said ends. Some of the common chemical modifications by which oligonucleotide and oligoribonucleotide molecules are modified include modification of part or all of the nucleotides with 2'-methoxy group (2'-O-methyl: 2'-OMe), 2'-O-methoxyethyl group (2'-MOE), and/or phosphorothioate bonds.

In particular, for the purposes of the invention, of special interest (and considered to be included within the modifications that give rise to antagonists included within the scope of the invention) are those modifications, valid for oligonucleotides and oligoribonucleotides that give rise to DNA and RNA analogs with increased resistance to hydrolysis, and which are generally modifications on the pentose, such as those resulting in: 2'-O-methyl-substituted (the 2'-methoxy modifications); 2'-O-methoxyethyl-substituted; LNAs (locked nucleic acids, in which the pentose moiety is modified with an extra bridge connecting the 2' oxygen and 4' carbon and locking the pentose in the 3'-endo conformation); BNAs (bridged nucleic acids); PMOs (nucleic acids wherein the ribose has been replaced by a morpholino group); or PNAs (peptide nucleic acids, wherein the pentose phosphate group is replaced by an amino acid residue, such that the nucleotide analog has as its backbone a structure of repeating N-(2-aminoethyl)-glycine units attached by peptide bonds). Recently, nucleotides with different modifications have also been used, such as CRNs (conformationally restricted nucleotides), in which the pentose moiety is locked in a conformation controlled by means of a chemical moiety that acts as a connector, a modification that is being used mainly to obtain antagomiRs with new properties, or cETs (cET = constrained ethyl).

Also included among the possible modifications that give rise to oligonucleotide or oligoribonucleotide analogs of the invention are modifications that give rise to phosphorothioate bonds, which are modifications that affect phosphate groups that are part of the "backbone" of the nucleotide chain, giving rise to the introduction of a sulfur atom replacing an oxygen atom of the phosphate group that is not acting as a bridge between nucleotides; these modifications make the bonds between nucleotides resistant to degradation by nucleases, so it is common to introduce them between the last 3-5 nucleotides located at the 5' or 3' ends of the antagonist oligonucleotide to inhibit degradation thereof by exonucleases, increasing stability. Phosphorothioate bonds also appear to increase binding to serum proteins, such as albumins, to improve the distribution of molecules containing them. Also included among the chemical modifications giving rise to oligonucleotide and oligoribonucleotide analogs of the invention is methylation at 5' of the nitrogenous base cytosine (C), which appears to increase the stability of the complexes formed with the target.

Other chemical modifications, which are also comprised within the possible modifications which give rise to oligonucleotide or oligoribonucleotide analogs, are also possible and known. As can be deduced from the definition of "molecules of an oligonucleotide/oligoribonucleotide nature" and of "oligonucleotide/oligoribonucleotide analogs", also comprised within the definition of oligonucleotide or oligoribonucleotide analogs are molecules in which some units have modifications and others do not, as well as hybrids between nucleic acid analogs and peptides or even hybrid molecules in which some of the nucleotide units are ribonucleotides (or analogs thereof) and others are deoxyribonucleotides (nucleotides in which the sugar is deoxyribose), as well as analogs of the latter, i.e., RNA-DNA hybrids and analogs thereof.

Preferably, the molecule of an oligonucleotide nature and/or an oligonucleotide analog is an antagonist of a human microRNA selected from the group of miR-100, miR-20, miR-222, miR-181, and miR-92, and wherein said antagonist is an antimiR, a blockmiR, or an miRNA sponge.

As used herein, "blockmiR" refers to small oligonucleotides with a special chemistry designed against the sequence that a particular miRNA, in this case miR-100-5p, miR-20a-5p, miR-222-3p, miR-181c-5p, and miR-92a-3p, recognizes in its target messenger RNA (mRNA), so each of them should only de-repress the effect of that miRNA on that transcript, with a very specific effect being expected. Therefore, they are designed to have a sequence that is complementary to that of a fragment of the sequence of an mRNA that serves as a binding site for an miRNA, such that they typically bind in the 3' untranslated region (3'-UTR) of an mRNA, i.e., in the area in which endogenous miRNAs typically bind.

In a preferred embodiment, the antagonist is an antimiR. AntimiRs are generally used to silence endogenous miRNAs. Therefore, as used herein, "antimiR", which is synonymous with "antagomiR", refers to small synthetic oligonucleotides, preferably synthetic oligoribonucleotides, that are complementary to a target miRNA, in this case miR-100-5p, miR-20a-5p, miR-222-3p, miR-181c-5p, and miR-92a-3p, and therefore act as antagonists of said miRNAs. Typically, antimiRs include chemical modifications such as 2-O-methyl groups, phosphorothioates, and conjugated fatty acids, preferably cholesterol.

As used herein, "miRNA sponge" refers to molecules the main constituent of which is oligonucleotide repeats arranged in tandem, with the particularity that each one of said oligonucleotides is itself or contains a binding site for binding to the miR-100-5p, miR-20a-5p, miR-222-3p, miR-181c-5p, and miR-92a-3p that it antagonizes.

To design antagonist molecules, it is important to take into account the existence of sufficient complementarity with the sequence of the target miR, in the case of antimiRs and miRNA sponges, or their target mRNA, in the case of blockmiRs, to which they must bind in order to actually produce the desired inhibition/antagonism effect. In that sense, examples of the "typical" complementarity between an antagonist and its target being 50% can be taken into account. Therefore, in a preferred embodiment, the antagonist of an oligonucleotide or oligoribonucleotide nature of the invention comprises a fragment of the sequence of nucleotide or ribonucleotide units, or analog units thereof, in which the sequence of the nitrogenous bases of the ribonucleotide or ribonucleotide analog units is at least 50%, 55%, 60%, 65%, 70%, 75%, 80%, 85%, 90%, 91%, 92%, 93%, 94%, 95%, 96%, 97%, 98%, 99%, or 99.5%, or 100% identical to the complementary sequence of the endogenous molecule with which it is to hybridize, i.e., to the sequences of miR-100-5p, miR-20a-5p, miR-222-3p, miR-181c-5p, and miR-92a-3p to which it must bind (in the case of antagomiRs and the repeat sequence of miRNA sponges) or the sequence of the fragment of the messenger mRNA (in the case of blockmiRs). In the context of the present invention, nitrogenous bases T and U are considered interchangeable, and therefore the sequences including T can also be considered sequences with U, and vice versa.

In the event that the antagonist of an oligonucleotide or oligoribonucleotide nature of the invention is an antimiR, in a preferred embodiment, said antimiR comprises a fragment of the sequence of nucleotide or ribonucleotide units, or analog units thereof, in which the sequence of the nitrogenous bases of the ribonucleotide or ribonucleotide analog units is at least 50%, 55%, 60%, 65%, 70%, 75%, 80%, 85%, 90%, 91%, 92%, 93%, 94%, 95%, 96%, 97%, 98%, 99%, or 99.5%, or 100% identical to the complementary sequence of any of sequences of human microRNAs SEQ ID NO: 1 (miR-100-5p), SEQ ID NO: 14 (miR-20a-5p), SEQ ID NO: 15 (miR-222-3p), SEQ ID NO: 16 (miR-181c-5p), or SEQ ID NO: 17 (miR-92a-3p).

Furthermore, endogenous miRNAs usually bind in the 3' untranslated region (3'-UTR) of the mRNA they regulate. However, the binding between the miRNA and its target mRNA does not have to be perfect and is mainly dominated by the so-called seed region of the miRNA. The seed region sequence is a conserved sequence that is mainly located at positions 2-8 of the 5' end of the miRNA. Even if the base pairing of the miRNA and its target mRNA does not perfectly match, with the "seed sequence" it has to perfectly match, i.e., 100%, complementary. In the case of miR-100-5p, its sequence corresponds to SEQ ID NO: 1, of which the seed region is made up of SEQ ID NO: 2. In the case of miR-20a-5p, its sequence corresponds to SEQ ID NO: 14, of which the seed region is made up of SEQ ID NO: 18. In the case of miR-222-3p, its sequence corresponds to SEQ ID NO: 15, of which the seed region is made up of SEQ ID NO: 19. In the case of miR-181c-5p, its sequence corresponds to SEQ ID NO: 16, of which the seed region is made up of SEQ ID NO: 20. In the case of miR-92a-3p, its sequence corresponds to SEQ ID NO: 17, of which the seed region is made up of SEQ ID NO: 21.

It can be deduced from the foregoing that when designing antagonists of human microRNAs miR-100-5p, miR-20a-5p, miR-222-3p, miR-181c-5p, and miR-92a-3p of an oligonucleotide or oligoribonucleotide nature or analogs thereof, it is advisable to consider the seed region thereof, and to include the region highly complementary to said seed region in the antagonist being designed. Therefore, in a preferred embodiment, the antagonist of an oligonucleotide or oligoribonucleotide nature of the invention is an antimiR comprising a fragment of the sequence of nucleotide or ribonucleotide units or analog units thereof, in which the sequence of the nitrogenous bases of the ribonucleotide or ribonucleotide analog units is at least 95%, 96%, 97%, 98%, 98%, 99%, or 99.5%, or 100% identical to the sequence complementary to the seed region of any of human microRNAs miR-100-5p, miR-20a-5p, miR-222-3p, miR-181c-5p, or miR-92a-3p, as determined by SEQ ID NOs: 2, 18, 19, 20, 20, or 21, respectively. Preferably, the identity to the sequence complementary to the seed region is 100%.

The length of the sequence of the antagonists of an oligonucleotide or oligoribonucleotide nature or analogs thereof is at least 8 nucleotides, without a maximum having been established. Preferably, the length of the sequence of said antagonists is at least 8, 9, 10, 11, 12, 13, 14, 15, 16, 17, 18, 19, 20, 21, 22, 23, 24, 25, 30, 35, 40, 45, or 50 nucleotides. In a preferred embodiment, the length of said antagonists is from 10-15, 10-20, 20-30, 30-40, or 40-50. In another preferred embodiment, the length of said antagonists is from 15-25 nucleotides. In one embodiment of the present invention, the antagonist is an antimiR comprising or consisting of a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to the complementary sequence of any of sequences SEQ ID NO: 1 (miR-100-5p), SEQ ID NO: 14 (miR-20a-5p), SEQ ID NO: 15 (miR-222-3p), SEQ ID NO: 16 (miR-181c-5p), or SEQ ID NO: 17 (miR-92a-3p), preferably SEQ ID NO: 1. In a preferred embodiment, the antagonist is an antimiR comprising or consisting of a region of at least 5, 6, 7, or 8 contiguous nucleotide or nucleotide analog units which is 100% identical to the sequence complementary to the seed region of any of human microRNAs as defined in SEQ ID NO: 2 (miR-100-5p seed), SEQ ID NO: 18 (miR-20a-5p seed), SEQ ID NO: 19 (miR-222-3p seed), SEQ ID NO: 20 (miR-181c-5p seed), or SEQ ID NO: 21 (miR-92a-3p seed), preferably SEQ ID NO: 2.

In another preferred embodiment, the antagonist is an antimiR comprising a first region of at least 5, 6, 7, or 8 contiguous nucleotide or nucleotide analog units which is 100% identical to the sequence complementary to the seed region of any of human microRNAs miR-100, miR-20, miR-222, miR-181, or miR-92, as defined in SEQ ID NO: 2 (miR-100-5p seed), SEQ ID NO: 18 (miR-20a-5p seed), SEQ ID NO: 19 (miR-222-3p seed), SEQ ID NO: 20 (miR-181c-5p seed), and SEQ ID NO: 21 (miR-92a-3p seed), and wherein said antagonist further comprises a second region of at least 10, 11, 12, 13, 14, 15, 16, 17, 18, 19, 20, 21, 22, or 23 contiguous nucleotide or nucleotide analog units adjacent to the first region, wherein said second region is at least 50%, 55%, 60%, 65%, 70%, 75%, 80%, 85%, 90%, 91%, 92%, 93%, 94%, 95%, 96%, 97%, 98%, 99%, or 99.5%, or 100% identical to the sequence complementary to any region present in SEQ ID NO: 1 (miR-100-5p), SEQ ID NO: 14 (miR-20a-5p), SEQ ID NO: 15 (miR-222-3p), SEQ ID NO: 16 (miR-181c-5p), or SEQ ID NO: 17 (miR-92a-3p), respectively.

As described in the examples, the authors of the present invention found that antagonists of human microRNAs miR-100-5p, miR-20a-5p, miR-222-3p, miR-181c-5p, and miR-92a-3p, are capable of increasing the levels of the MBNL1 protein and the expression levels of the MBNL2 protein in a muscle cell. Therefore, a series of antimiRs specific against said miRs were designed. Human microRNA sequences, from 5' to 3', are (the seed region is underlined):
hsa-miR-100-5p (SEQ ID NO: 1): AACCCGUAGAUCCGAACUUGUG
hsa-miR-20a-5p (SEQ ID NO: 14): UAAAGUGCUUAUAGUGCAGGUAG
hsa-miR-181c-5p (SEQ ID NO: 16): AACAUUCAACCUGUCGGUGAGU
hsa-miR-222-3p (SEQ ID NO: 15): AGCUACAUCUGGCUACUGGGU
hsa-miR-92a-3p (SEQ ID NO: 17): UAUUGCACUUGUCCCGGCCUGU

The sequences of the designed antagomiRs are, from 5' to 3':
AntagomiR-100-5p (SEQ ID NO: 3): CACAAGTTCGGATCTACGGGTT
AntagomiR-20a-5p (SEQ ID NO: 4): CTACCTGCACTATAAGCACTTTA
AntagomiR-181c-5p (SEQ ID NO: 5): ACTCACCGACAGGTTGAATGTT
AntagomiR-222-3p (SEQ ID NO: 6): ACCCAGTAGCCAGATGTAGCT
AntagomiR-92a-3p (SEQ ID NO: 7): ACAGGCCGGGACAAGTGCAATA

In the case of antimiRs, the antagonist of an oligonucleotide or oligoribonucleotide nature of the invention comprises or consists of a fragment of the sequence of nucleotide or ribonucleotide units, or analog units thereof, in which the sequence of the nitrogenous bases of the nucleotide or nucleotide analog units is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of sequences SEQ ID NO: 3, 4, 5, 6, or 7, preferably SEQ ID NO: 3. In a preferred embodiment, the antagonist consists of sequence SEQ ID NO: 3, 4, 5, 6, or 7, preferably SEQ ID NO: 3.

In one embodiment of the present invention, the antagonist is an antimiR comprising or consisting of a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any region present in any of sequences SEQ ID NO: 3, 4, 5, 6, or 7, preferably SEQ ID NO: 3.

Furthermore, the developed antagomiRs can have certain chemical modifications that are common in this type of oligonucleotide or oligoribonucleotide analogs, among which are the following:
- 2'-O-methyl (2'-methoxy) modifications on all the pentose moieties,
- the replacement of some phosphate bonds between monomeric nucleotide analog units with phosphorothioate bonds, and
- the incorporation of fatty acid molecules at one end of the molecule, preferably at the 3' end.

In this way, in a preferred embodiment, the antagonist has at least one chemical modification in its structure, wherein:
i. said antagonist comprises a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any region present in any of sequences SEQ ID NO: 3, 4, 5, 6, or 7, preferably SEQ ID NO: 3,
ii. at least one of the nucleotide or nucleotide analog units making up said antagonist is a nucleotide analog having one or more chemical modifications in the pentose moiety, preferably ribose, in the phosphate bond, or in both, and
iii. optionally, said antagonist has at the 5' end and/or at the 3' end one or more additional molecules, preferably one or more molecules which are not deoxyribonucleotide or ribonucleotide derivatives.

In another preferred embodiment, the antagonist has at least one chemical modification in its structure, wherein:
i. said antagonist comprises a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any region present in any of sequences SEQ ID NO: 3, 4, 5, 6, or 7, preferably SEQ ID NO: 3,
ii. at least one of the nucleotide or nucleotide analog units making up said antagonist is a nucleotide analog having one or more chemical modifications selected from the group of: methylations, phosphorothioate bonds, LNAs, BNAs, constrained ethyl (cEt) nucleosides, PMOs, PNAs, 2'-O-methyl-substituted, 2'-O-methoxyethyl-substituted, 2'-fluoro, and
iii. optionally, said antagonist has at the 5' end and/or at the 3' end one or more additional molecules, preferably one or more molecules which are not deoxyribonucleotide or ribonucleotide derivatives.

In another preferred embodiment, the antagonist has at least one chemical modification in its structure, wherein:
i. said antagonist comprises a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any region present in any of sequences SEQ ID NO: 3, 4, 5, 6, or 7, preferably SEQ ID NO: 3,
ii. at least one of the nucleotide or nucleotide analog units making up said antagonist is a nucleotide analog having one or more chemical modifications in the ribose moiety, in the phosphate bond, or in both, preferably selected from the group of: methylations, phosphorothioate bonds, LNAs, BNAs, constrained ethyl (cEt) nucleosides, PMOs, PNAs, 2'-O-methyl-substituted, 2'-O-methoxyethyl-substituted, 2'-fluoro, and
iii. optionally, said antagonist has at the 5' end and/or at the 3' end one or more additional molecules, preferably one or more molecules which are not deoxyribonucleotide or ribonucleotide derivatives, preferably cholesterol.

This therefore gives rise to the following oligonucleotide or oligoribonucleotide analogs antagonists of human microRNAs miR-100, miR-20, miR-222, miR-181, and miR-92:
AntagomiR-100-5p (SEQ ID NO: 8): **C*A*CAAGTTCGGATCTACGG*G*T*T***
AntagomiR-20a-5p (SEQ ID NO: 9): **C*T*ACCTGCACTATAAGCACT*T*T*A***
AntagomiR-181c-5p (SEQ ID NO: 10): **A*C*TCACCGACAGGTTGAAT*G*T*T***
AntagomiR-222-3p (SEQ ID NO: 11): **A*C*CCAGTAGCCAGATGTA*G*C*T***
AntagomiR-92a-3p (SEQ ID NO: 12): **A*C*AGGCCGGGACAAGTGCA*A*T*A***
where:
- an asterisk (*) indicates the presence of a phosphorothioate bond,
- A, C, T, G in bold indicates that said nucleotide is modified with 2'-O-methoxyethyl, and
- optionally, a fatty acid is conjugated at the 3' and/or 5' ends of the molecule.

Therefore, all the nitrogenous bases of sequences SEQ ID NO: 8, 9, 10, 11, and 12 are modified with 2'-O-methoxyethyl (2'-OMe).

In a preferred aspect, sequences SEQ ID NO: 8, 9, 10, 11, and 12 have a fatty acid, preferably cholesterol, conjugated at their 3' end.
AntagomiR-100-5p (SEQ ID NO: 13): **C*A*CAAGTTCGGATCTACGG*G*T*T***-Chol
AntagomiR-20a-5p (SEQ ID NO: 22): **C*T*ACCTGCACTATAAGCACT*T*T*A***-Chol
AntagomiR-181c-5p (SEQ ID NO: 23): **A*C*TCACCGACAGGTTGAAT*G*T*T***-Chol
AntagomiR-222-3p (SEQ ID NO: 24): **A*C*CCAGTAGCCAGATGTA*G*C*T***-Chol
AntagomiR-92a-3p (SEQ ID NO: 25): **A*C*AGGCCGGGACAAGTGCA*A*T*A***-Chol
where:
- an asterisk (*) indicates the presence of a phosphorothioate bond,
- A, C, T, G in bold indicates that said nucleotide is modified with 2'-O-methoxyethyl, and
- "-Chol" indicates that the oligonucleotide is conjugated to cholesterol at its 3' end.

Therefore, all the nitrogenous bases of sequences SEQ ID NO: 13, 22, 23, 24, and 25 are modified with 2'-O-methoxyethyl (2'-OMe) and conjugated a cholesterol.

In a preferred embodiment, the antagonist of an oligonucleotide or oligoribonucleotide nature of the invention is an antimiR comprising or consisting of a fragment of the sequence of nucleotide or ribonucleotide units, or analog units thereof, in which the sequence of the nitrogenous bases of the nucleotide or nucleotide analog units is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of the sequences SEQ ID NO: 8, 9, 10, 11, or 12, preferably SEQ ID NO: 8. In a preferred embodiment, the antagonist consists of SEQ ID NO: 8, 9, 10, 11, or 12, preferably SEQ ID NO: 8.

In one embodiment of the present invention, the antagonist is an antimiR comprising or consisting of a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of the sequences SEQ ID NO: 8, 9, 10, 11, or 12, preferably SEQ ID NO: 8.

In a preferred embodiment, the antagonist of an oligonucleotide or oligoribonucleotide nature of the invention is an antimiR comprising or consisting of a fragment of the sequence of nucleotide or ribonucleotide units, or analog units thereof, in which the sequence of the nitrogenous bases of the nucleotide or nucleotide analog units is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of the sequences SEQ ID NO: 13, 22, 23, 24, 25 or 26-30, preferably SEQ ID NO: 13; wherein a cholesterol molecule is conjugated at the 3' end of the antagonist. In a preferred embodiment, the antagonist consists of any of the sequences SEQ ID NO: 13, 22, 23, 24, 25 or 26-30, preferably SEQ ID NO: 13, wherein a cholesterol molecule is conjugated at the 3' end of said antagonist.

In one embodiment of the present invention, the antagonist is an antimiR comprising or consisting of a region of at least 7, 8, 9, 10, 11, 12, 13, 14, 15, 20, 21, 22, or 23, preferably between 20 and 23, contiguous nucleotide or nucleotide analog units which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of the sequences SEQ ID NO: 13, 22, 23, 24, 25 or 26-30, preferably SEQ ID NO: 13, wherein a cholesterol molecule is conjugated at the 3' end of said antagonist.

A possible embodiment of the invention can be considered to be a molecule of an oligonucleotide nature and/or an oligonucleotide analog which is an antagonist of a human microRNA, wherein said human microRNA is selected from the group of miR-100, miR-20, miR-222, miR-181, and miR-92, or a mixture of two or more of said molecules, which is capable of increasing the intracellular levels of MBNL1 and/or MBNL2 proteins or the levels of their transcripts in a muscle cell. Said muscle cell can be one or more cells of the skeletal muscle tissue, cardiac muscle tissue, or smooth muscle tissue, or the precursors thereof. In a preferred embodiment, said muscle cell is a cardiomyocyte, myoblast, myocyte, striated muscle cell, or smooth muscle cell. In another preferred embodiment, the antagonist is capable of increasing the levels of MBNL1 and/or MBNL2 proteins or the intracellular levels of their transcripts in a cell found in one or more organs selected from the group of brain, cerebellum, hippocampus, or other organ of the central nervous system, skeletal muscle, heart, adipose tissue, kidney, liver and biliary system, lung, pharynx, nasopharynx, nose, placenta, spleen, testicle, uterus, gastrointestinal tract, breast, bladder, prostate, skin, or in one or more cells from a primary culture of one of said organs or from an established cell line derived from one of said organs (including induced pluripotent stem cells, known as iPSCs) or stem cells from one of said organs.

In a second aspect, the present invention relates to a composition comprising at least one antagonist as defined in the first aspect or in any of its embodiments, and at least one pharmaceutically acceptable excipient. Furthermore, the antagonist can be delivered in a vector. Said vector can be an expression vector. The vector can be selected from the list including DNA, RNA, viral, liposome, or exosome vectors. The present invention thereby also includes any delivery or expression means comprising the inhibitors or antagonists defined in the first aspect or in any of its embodiments.

It is common to prepare compositions comprising more than one active ingredient. For example, it would be possible to prepare a composition comprising the combination of the antagonists of the present invention with other antagonists of other human microRNAs which also intervene or have an effect on the production of MBNL proteins. In that sense, in a preferred embodiment of the second aspect, the composition comprises a combination of antagonists, which include the antagonist as defined in the first aspect or in any of its embodiments. In a preferred aspect, the composition according to the second aspect comprises at least one antagonist as defined in the first aspect or in any of its embodiments, and at least one antagonist of human microRNA-218-5p or human microRNA-23b-3p. Human microRNA-218-5p or human microRNA-23b-3p are defined in patent ES 2 659 845 A1. In another embodiment, the composition according to the second aspect comprises at least one antagonist as defined in the first aspect or in any of its embodiments, and other molecules of a nucleotide nature, preferably antisense oligonucleotides (ASOs).

Preferably, the composition according to the second aspect comprises at least one antagonist against human microRNA miR-100, preferably an antagonist comprising or consisting of SEQ ID NO: 8, 13, or 26, or an antagonist the sequence of which is at least 50%, 55%, 60%, 65%, 70%, 75%, 80%, 85%, 90%, 91%, 92%, 93%, 94%, 95%, 96%, 97%, 98%, 99%, or 99.5%, or 100% identical to any of the sequences SEQ ID NO: 8, 13, or 26.

For clinical application, the compositions of the present invention, which will then be considered pharmaceutical compositions of the present invention, can be prepared in a form suitable for the desired application. As disclosed in publications also relating to the clinical application of inhibitors/antagonists of microRNAs, such as international application WO2012148373A1, this will generally involve the preparation of compositions that are essentially free of pyrogens, as well as other impurities that may be harmful to humans or animals. Said international application WO2012148373A1 relates to compounds analogous to those of the present invention and to therapeutic applications thereof, whereby information on forms of preparation and presentation of pharmaceutical compositions, possible suitable administration vehicles, or forms and routes of administration may be considered applicable to the present invention and may be taken as a reference for the compositions of the present invention.

Colloidal dispersion systems, such as macromolecule complexes, nanocapsules, microspheres, beads, and lipid-based systems including oil-in-water emulsions, micelles, mixed micelles, and liposomes, can be used as delivery vehicles for the inhibitors/antagonists of the present invention, with which the pharmaceutical composition of the invention is formed, but the possibility for the antagonists to be administered without the aid of other pharmacological agents is also included.

Another possibility, as already discussed, is to prepare the pharmaceutical compositions of the invention using suitable salts and buffers to make the delivery vehicles stable and aid in capture by the target cells. The compositions of the present invention may be aqueous compositions comprising an effective amount of the delivery vehicle and comprising either the molecules of an oligonucleotide nature of the invention, independently or forming liposomes or other complexes, or expression vectors thereof, dissolved or dispersed in a pharmaceutically acceptable vehicle or aqueous medium. The expressions "pharmaceutically acceptable" or "pharmacologically acceptable" refer to molecular entities and compositions that do not produce adverse reactions, allergic or otherwise, when administered to an animal or human being. As used in the present specification, "pharmaceutically acceptable vehicle" includes solvents, buffers, solutions, dispersion media, coatings, antibacterial and antifungal agents, isotonic and absorption retarding agents, and the like, acceptable for use in formulating pharmaceuticals, such as pharmaceuticals suitable for administration to humans. The use of such media and agents for pharmaceutically active substances is well known in the art. Except to the extent that any conventional medium or agent is incompatible with the active ingredients of the present invention, the use thereof in the pharmaceutical compositions of the invention is contemplated. Supplemental active ingredients may also be incorporated in the compositions, provided that they do not inactivate the molecules of the present invention or their expression vectors.

The active compositions of the present invention can be administered by any of the common routes, provided that the target tissue is available through that route. This includes oral, nasal, or buccal routes and also, preferably, administration may be by intradermal, subcutaneous, intramuscular, intraperitoneal, or intravenous routes.

Suitable pharmaceutical forms for injectable use include, for example, sterile aqueous solutions or sterile dispersions and powders for the extemporaneous preparation of sterile injectable solutions or dispersions. Generally, these preparations are sterile and fluid to the extent that there is easy injectability. The preparations must be stable under manufacturing and storage conditions and must be preserved against the contaminating action of microorganisms, such as bacteria and fungi. Suitable solvents or dispersion media may contain, for example, water, ethanol, polyol (e.g., glycerol, propylene glycol, and liquid polyethylene glycol, and the like), suitable mixtures thereof, and vegetable oils. Suitable fluidity can be maintained, for example, by means of using a coating, such as lecithin, by means of maintaining the required particle size in the case of dispersion, and by means of using surfactants. Prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be caused by use in the compositions of agents that delay absorption, for example, aluminum monostearate and gelatin.

Sterile injectable solutions can be prepared by incorporating the active compounds in a suitable amount in a solvent along with any other ingredients (e.g., as specified above) as desired, followed by sterilization by filtration. Generally, dispersions are prepared by incorporating the various sterilized active ingredients in a sterile vehicle containing the basic dispersion medium and the other desired ingredients, e.g., as specified above. In the case of sterile powders for the preparation of sterile injectable solutions, preferred methods of preparation include vacuum drying and lyophilization, techniques that produce a powder of the active ingredient(s) plus any additional desired ingredients from a solution thereof previously sterilized by filtration.

The compositions of the present invention can generally be formulated in a neutral or salt form. Pharmaceutically acceptable salts include, for example, acid addition salts (formed with the free amino groups of the protein) derived from inorganic acids (e.g., hydrochloric or phosphoric acid), or from organic acids (e.g., acetic, oxalic, tartaric, mandelic acids), and the like. Salts formed with the free carboxyl groups of the protein can also be derived from inorganic bases (e.g., sodium, potassium, ammonium, calcium, or ferric hydroxides) or organic bases (e.g., isopropylamine, trimethylamine, histidine, procaine, and the like).

In any case, it is recommended that the preparation of the compositions of the present invention follow practices that ensure a minimum quality for use in humans, such as those contained in the Guide to Good Manufacturing Practice for Active Pharmaceutical Ingredients of Working Group Q7 of the International Conference on Harmonization of Technical Requirements for Registration of Pharmaceuticals for Human Use.

Preferably, other quality guidelines from the same source, such as Q8 on Pharmaceutical Development or Q10 on the Pharmaceutical Quality System, are also taken into account.

After formulation, the solutions are preferably administered in a form compatible with the dosage formulation and in such an amount as to be therapeutically effective. The formulations can be readily administered in a variety of dosage forms such as injectable solutions, drug release capsules, and the like. For parenteral administration in an aqueous solution, for example, the solution is generally suitably buffered and the liquid diluent must first be made isotonic, for example, with sufficient saline or glucose. Such aqueous solutions can be used, for example, for intravenous, intramuscular, subcutaneous, and intraperitoneal administration. Preferably, sterile aqueous media are used, as is known to those skilled in the art, selected particularly in light of the present description. Preferably, chemical buffers, which are buffering or regulating solutions that maintain the pH of the composition stable, are used. Examples of buffers are PBS. By way of illustration, a single dose can be dissolved in 1 ml of isotonic NaCl solution and added to 1000 ml of hypodermoclysis fluid or injected into the proposed infusion site (see, for example, "Remington Pharmaceutical Sciences" 15th edition, pages 1035-1038 and 1570-1580). Some variations in dosage will necessarily occur depending on the condition of the subject being treated. The person responsible for administration will, in any case, determine the suitable dosage for the individual subject. Moreover, for human administration, preparations must meet biological standards of sterility, pyrogenicity, general safety, and purity as required, for example, by the ICH Quality Guidelines cited above or by FDA regulations.

Furthermore, the significant increase of MBNL1 and/or MBNL2 proteins demonstrated in the present invention leads to consider the possible use of these antagonists as a drug. Therefore, a **third aspect** of the invention relates to the antagonist according to the first aspect or any of its embodiments, or to the composition according to the second aspect or any of its embodiments, for use thereof as a drug. In a preferred embodiment, the use is as a treatment for myotonic dystrophy, preferably myotonic dystrophy type 1.

The administration of the antagonist by means of a possible expression vector expressing same can allow directing the expression to a specific tissue or group of tissues depending on the tropism of the base vector itself and/or by means of the choice of control elements that result in the expression of the coding sequence linked to same only in specific tissues. As for the organs or tissues to be treated, preferably they are those tissues or organs affected by DM1, in particular those selected from the group of skeletal muscle, cardiac muscle, smooth muscles, diaphragm, respiratory muscles, or pharyngeal muscles or stem cells of one or more of said organs.

Furthermore, particular dosage forms may favor greater access to one or more other organs. The concentration assays performed in the examples show that the antagomiRs, at different doses, are able to increase the expression and concentration of MBNL1 and/or MBNL2 proteins, which supports their use for the preparation of a drug for the treatment of myotonic dystrophy 1, in particular to palliate symptoms of the disease, especially symptoms corresponding to muscle dysfunction. In one possible embodiment, the pharmaceutical composition comprises an effective dose of an inhibitor or antagonist of at least one of the human microRNAs miR-100, miR-20, miR-222, miR-181, and miR-92. More preferably, the inhibitor(s)/antagonist(s) will be present at a concentration that allows the administration of a therapeutically effective dose.

An "effective dose" or "therapeutically effective dose" is an amount sufficient to effect a beneficial or desired clinical outcome. Furthermore, the effective dose may vary in organisms of different species and/or sizes. Thus, an effective dose of an inhibitor/antagonist of a microRNA in rodents, in accordance with previous results obtained with molecules targeting other microRNAs, can be from about 1 mg/kg to about 100 mg/kg, about 2.5 mg/kg to about 50 mg/kg, or about 5 mg/kg to about 25 mg/kg. It should be noted that the therapeutic doses tested in mice must be converted to their human equivalent. For this purpose, the person skilled in the art may consult the well-known common practice guide for dose conversion between animals and human, see, for example, Nair AB, Jacob S. A simple practice guide for dose conversion between animals and human. J Basic Clin Pharma 2016;7:27-31. Briefly, therapeutic doses in mouse models can be converted to humans by dividing by about 12.3, although a corresponding clinical trial would have to be performed. Calculating and obtaining an effective dose for humans is within routine practice and therefore within the knowledge of one skilled in the art. Furthermore, the precise determination of what is considered an effective dose may be based on individual factors for each patient, including their size, age, and the nature of the inhibitor or antagonist (e.g., whether it is an expression construct, an antagomiR or antimiR oligoribonucleotide analog). Therefore, dosages can be readily determined by those skilled in the art from this description and knowledge of the art. It may be necessary or desirable to administer multiple doses to the subject during a particular treatment period, administering doses daily, weekly, monthly, every two months, every three months, or every six months. In certain embodiments, the subject receives an initial dose at a first time point that is greater than one or more subsequent or maintenance doses.

Given the stability of antagomiRs, it is possible to consider their administration to humans directly, for example by subcutaneous or systemic route, preferably by intravenous route, for example dissolved or suspended in a pharmaceutically acceptable vehicle, such as water or some aqueous solution such as, for example, saline or phosphate buffer.

Each embodiment described here is contemplated as being applicable to each of the other described embodiments. Therefore, all the combinations of the various elements described here are within the scope of the invention.

The following clauses also are included in the present invention:
1. An antagonist of human microRNA miR-100 capable of increasing the intracellular levels of MBNL1 and/or MBNL2 proteins.
2. The antagonist of human microRNA miR-100 according to clause 1, wherein the intracellular levels of MBNL1 and/or MBNL2 proteins are increased in a muscle cell, preferably in a muscle cell having a muscular dystrophy phenotype, preferably myotonic dystrophy type 1.
3. The antagonist of human microRNA miR-100 according to any of clauses 1 or 2, wherein said antagonist is a molecule of an oligonucleotide nature and/or an oligonucleotide analog.
4. The antagonist of human microRNA miR-100 according to any of clauses 1 to 3, wherein said antagonist is an antimiR, a blockmiR, or a microRNA sponge.
5. The antagonist of human microRNA miR-100 according to any of clauses 1 to 4, wherein said antagonist comprises a region which is at least 85% identical to the complementary sequence of SEQ ID NO: 1.
6. The antagonist of human microRNA miR-100 according to any of clauses 1 to 5, wherein said antagonist comprises a region of at least 7 contiguous nucleotide or nucleotide analog units which is 100% identical to the sequence complementary to the seed region of human microRNA miR-100 as defined in SEQ ID NO: 2.
7. The antagonist of human microRNA miR-100 according to any of clauses 1 to 6, wherein said antagonist comprises a first region of at least 7 contiguous nucleotide or nucleotide analog units which is 100% identical to the sequence complementary to the seed region of human microRNA miR-100 as defined in SEQ ID NO: 2, and wherein said antagonist further comprises a second region of at least 7 contiguous nucleotide or nucleotide analog units adjacent to the first region, wherein said second region is at least 90% identical to the sequence complementary to any region present in SEQ ID NO: 1.
8. The antagonist of human microRNA miR-100 according to any of clauses 1 to 9, wherein said antagonist comprises a nucleotide or nucleotide analog region which is at least 90% identical to SEQ ID NO: 3.
9. The antagonist of human microRNA miR-100 according to any of clauses 1 to 8, wherein:
   i) said antagonist comprises a region which is at least 95% identical to SEQ ID NO: 3,
   ii) at least one of the nucleotide or nucleotide analog units making up said antagonist is a nucleotide analog having one or more chemical modifications in the ribose moiety, in the phosphate bond, or in both, and
   iii) optionally, said antagonist has at the 5' end and/or at the 3' end one or more additional molecules, preferably cholesterol.
10. The antagonist of human microRNA miR-100 according to any of clauses 1 to 9, wherein said antagonist comprises a nucleotide or nucleotide analog region which is at least 95% identical to any of the sequences SEQ ID NO: 8 or 13.
11. The antagonist of human microRNA miR-100 according to any of clauses 9 or 10, wherein said antagonist consists of SEQ ID NO: 8 or SEQ ID NO: 13.
12. A composition comprising at least one antagonist of human microRNA miR-100 as defined in any of clauses 1 to 11.
13. The composition according to clause 12, wherein said composition is a pharmaceutical composition further comprising a pharmaceutically acceptable vehicle and/or one or more pharmaceutically acceptable excipients.
14. The antagonist according to any of clauses 1 to 11, or composition according to any of clauses 12 or 13, for use thereof as a drug.
15. The antagonist according to any of clauses 1 to 11, or composition according to any of clauses 12 or 13, for use thereof in the treatment of myotonic dystrophy, preferably myotonic dystrophy type 1.

### SEQUENCE LISTING

Sequence of human microRNA miR-100-5p: SEQ ID NO 1:
AACCCGUAGAUCCGAACUUGUG

Seed sequence of human microRNA miR-100-5p: SEQ ID NO 2: CCCGUAG

Sequence of antagonist hsa-miR-100: SEQ ID NO 3:
CACAAGTTCGGATCTACGGGTT

Sequence of antagonist hsa-miR-20a-5p: SEQ ID NO 4:
CTACCTGCACTATAAGCACTTTA

Sequence of antagonist hsa-miR-181c-5p: SEQ ID NO 5:
ACTCACCGACAGGTTGAATGTT

Sequence of antagonist hsa-miR-222-3p: SEQ ID NO 6:
ACCCAGTAGCCAGATGTAGCT

Sequence of antagonist hsa-miR-92a-3p: SEQ ID NO 7:
ACAGGCCGGGACAAGTGCAATA

Sequence of antagonist hsa-miR-100-5p with modifications: SEQ ID NO 8:
**C*A*CAAGTTCGGATCTACGG*G*T*T***

Sequence of antagonist hsa-miR-20a-5p with modifications: SEQ ID NO 9:
**C*T*ACCTGCACTATAAGCACT*T*T*A***

Sequence of antagonist hsa-miR-181c-5p with modifications: SEQ ID NO 10:
**A*C*TCACCGACAGGTTGAAT*G*T*T***

Sequence of antagonist hsa-miR-222-3p with modifications: SEQ ID NO 11:
**A*C*CCAGTAGCCAGATGTA*G*C*T***

Sequence of antagonist hsa-miR-92a-3p with modifications: SEQ ID NO 12:
**A*C*AGGCCGGGACAAGTGCA*A*T*A*** where:
- an asterisk (*) indicates the presence of a phosphorothioate bond,
- A, C, T, G in bold indicates that said nucleotide is modified with 2'-OMe, and
- optionally, a fatty acid is conjugated at the 3' and/or 5' ends of the molecule.

Sequence of antagonist hsa-miR-100-5p with modifications: SEQ ID NO 13:
**C*A*CAAGTTCGGATCTACGG*G*T*T***-Chol

Sequence of antagonist hsa-miR-20a-5p with modifications: SEQ ID NO 22:
**C*T*ACCTGCACTATAAGCACT*T*T*A***-Chol

Sequence of antagonist hsa-miR-181c-5p with modifications: SEQ ID NO 23:
**A*C*TCACCGACAGGTTGAAT*G*T*T***-Chol

Sequence of antagonist hsa-miR-222-3p with modifications: SEQ ID NO 24:
**A*C*CCAGTAGCCAGATGTA*G*C*T***-Chol

Sequence of antagonist hsa-miR-92a-3p with modifications: SEQ ID NO 25:
**A*C*AGGCCGGGACAAGTGCA*A*T*A***-Chol
where:
- an asterisk (*) indicates the presence of a phosphorothioate bond,
- A, C, T, G in bold indicates that said nucleotide is modified with 2'-OMe, and
- -Chol indicates that the oligonucleotide is conjugated to cholesterol.

Sequence of human microRNA miR-20a-5p: SEQ ID NO 14:
UAAAGUGCUUAUAGUGCAGGUAG

Sequence of human microRNA miR-222-3p: SEQ ID NO 15:
AGCUACAUCUGGCUACUGGGU

Sequence of human microRNA miR-181c-5p: SEQ ID NO 16:
AACAUUCAACCUGUCGGUGAGU

Sequence of human microRNA miR-92a-3p: SEQ ID NO 17:
UAUUGCACUUGUCCCGGCCUGU

Seed sequence of human microRNA miR-20a-5p SEQ ID NO 18: AAGUGCU

Seed sequence of human microRNA miR-222-3p: SEQ ID NO 19: CUACAUC

Seed sequence of human microRNA miR-181c-5p: SEQ ID NO 20: CAUUCAA

Seed sequence of human microRNA miR-92a-3p: SEQ ID NO 21: UUGCACU

**Sequence of antagonist hsa-miR-100-5p without modifications: SEQ ID NO 26:**
CACAAGTTCGGATCTACGGGTT

**Sequence of antagonist hsa-miR-20a-5p without modifications: SEQ ID NO 27:**
CTACCTGCACTATAAGCACTTTA

**Sequence of antagonist hsa-miR-181c-5p without modifications: SEQ ID NO 28:**
ACTCACCGACAGGTTGAATGTT

**Sequence of antagonist hsa-miR-222-3p without modifications: SEQ ID NO 29:**
ACCCAGTAGCCAGATGTAGCT

**Sequence of antagonist hsa-miR-92a-3p without modifications: SEQ ID NO 30:**
ACAGGCCGGGACAAGTGCAATA

The invention will now be illustrated in further detail with the help of the examples and figures which are shown below.

### EXAMPLES

### EXAMPLE 1: Screening based on mimetic microRNA libraries (SureFIND Transcriptome PCR Array, Qiagen).

This is a method for the identification of regulators of gene expression.

In this study, the "Cancer miRNA SureFind Transcriptome PCR Array" kit was used to identify potential miRNA regulators of MBNL1 and 2. These arrays include cDNA from HeLa cells treated with defined miRNA mimetics in 96-well plate format (90 different microRNAs and 6 controls) in which the target mRNAs of the miRNAs are expected to be detected significantly reduced or increased by real-time PCR. Multiplex qPCR assay was performed using commercial Taqman probes (QuantiFast Probe PCR Kits, Qiagen) to quantify the expression of MBNL1 and 2 (genes of interest, labeled with FAM) and GADPH (as reference endogenous expression, labeled with MAX). Changes in the expression of MBNL1 and 2 as a result of treatment with each specific mimetic microRNA were calculated with respect to the mimetic negative control (a microRNA that does not exist in nature) and normalized with respect to GADPH. The observed changes were plotted as log2 and subjected to ^{ΔΔCt} (MAD) statistical analysis for the selection of positive candidate miRNAs (Figure 1).

### Bioinformatic 3'UTR binding predictions

Starting from those microRNAs that had repressor activity on MBNL1 and MBNL2 at the messenger level, the inventors searched for possible targets of these microRNAs that bind directly to the 3'UTR of MBNL1 and MBNL2. For this purpose, 2 databases were used, mirDIP and miRecords, which collect information from a total of 9 prediction programs including TargetScan and miRanda, providing information on the existence of targets of a specific microRNA in the transcripts of a given gene suggesting direct regulation.

### Cell culture toxicity assay (TC50)

DM1 fibroblasts were cultured in Dulbecco's Modified Eagle Medium-high glucose (DMEM 4500 mg/l, Gibco) supplemented with 1% penicillin/streptomycin and 10% inactivated fetal bovine serum in cell culture bottles. Given their adherent growth, for their passage, they were washed with PBS and trypsinized for 2 min at 37°C, then fresh medium was added to inhibit the action of trypsin.

Cells for this assay were seeded at a density of 10⁵ cells/ml in 96-well plates (10,000 cells per well). The plate was seeded according to the following template. In the case of column 1 (blank), no cells were seeded, as this column is the target of the colorimetry analysis. The rest of the plate was seeded with DM1 fibroblasts, which were subsequently treated as described below. About 16 hours after cell seeding and with the cells at 80% confluence, the cells were transfected with the commercial antagomiRs provided by Biomers (antagomiR-miR-100-5p (SEQ ID NO: 3): CACAAGTTCGGATCTACGGGTT, antagomiR-20a-5p (SEQ ID NO: 4): CTACCTGCACTATAAGCACTTTA, antagomiR-181c-5p (SEQ ID NO: 5): ACTCACCGACGACAGGTTGAATGTT, antagomiR-222-3p (SEQ ID NO: 6): ACCCAGTAGCCAGATGTAGCT and antagomiR-miR-92a-3p (SEQ ID NO: 7): ACAGGCCCCGGGGGACAAGTGCAATA) using X-tremeGENE HP reagent (Roche) according to the manufacturer's instructions.

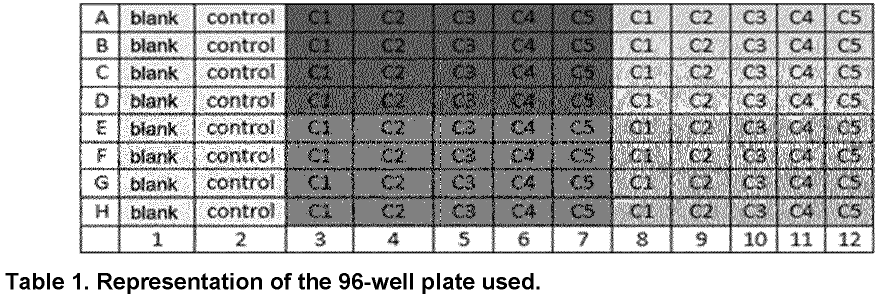

All the antagomiRs were transfected into patient fibroblasts (Arandel *et al.* 2017) using increasing amounts of 10 nM, 50 nM, 200 nM, and 1000 nM, and 5000 nM (C1, C2, C3, C4, and C5, respectively), and as a control only transfection reagent but no antagomiR was introduced, the transfection medium was left together with the cells for 4 hours, and after this time it was changed to transdifferentiation medium. To transdifferentiate fibroblasts to myoblasts, MyoD expression was induced. To that end, the complete medium was replaced with muscle differentiation medium (MDM) consisting of DMEM supplemented with 1% P/S, 2% horse serum (Gibco), 0.1 mg/ml apotransferrin, 0.01 mg/ml insulin, and 0.02 mg/ml doxycycline (Sigma) for 60 h.

After 72 h, the transdifferentiation medium was replaced with 100 µl of fresh medium in all wells of the plate including column 1, and 20 µl of the MTS/PMS solution (CellTiter 96^{®} Aqueous Non-Radioactive Cell Proliferation Assay Kit) were added to each well and incubated for 2 h at 37°C. After the incubation time, the colorimetric assay was read in the Infinite 200 PRO Microplate Reader, Tecan, following the manufacturer's instructions. The data obtained with the reader was processed and analyzed in order to obtain the TC50 which allows knowing the amount of antagomiR to be used for it not to be toxic in the cell model (Figure 2).

### Assay of the activity of antagomiR on MBNL1 protein (EC50)

Fibroblasts from DM1 patients and healthy controls were cultured in Dulbecco's Modified Eagle Medium-high glucose (DMEM 4500 mg/l, Gibco) supplemented with 1% P/S and 10% inactivated fetal bovine serum in cell culture bottles. The cells were seeded in 6-well plates, at a density of 100,000 cells/well by introducing 2.5 ml of cells in each well. About 16 hours after cell seeding and with the cells at 80% confluence, the cells were transfected with the commercial antagomiRs provided by Biomers (antagomiR-miR-100-5p (SEQ ID NO: 3): CACAAGTTCGGATCTACGGGTT, antagomiR-20a-5p (SEQ ID NO: 4): CTACCTGCACTATAAGCACTTTA, antagomiR-181c-5p (SEQ ID NO: 11): CTCACCGACGACAGGTTGAATGTT, antagomiR-222-3p (SEQ ID NO: 6): ACCCAGTAGCCAGATGTAGCT and antagomiR-miR-92a-3p (SEQ ID NO: 7): ACAGGCCCCGGGGGACAAGTGCAATA), using X-tremeGENE HP reagent (Roche).

The antagomiRs were transfected into patient fibroblasts using increasing amounts of 10 nM, 50 nM, 200 nM, 1000 nM, and 5000 nM. As a control only transfection reagent but no antagomiRs were introduced into both healthy and DM1 cells. Subsequently the transfection medium was left together with the cells for 4 hours and after this time it was changed to transdifferentiation medium (DMEM supplemented with 1% P/S, 2% horse serum (Gibco), 0.1 mg/ml apotransferrin, 0.01 mg/ml insulin, and 0.02 mg/ml doxycycline (Sigma)). Fibroblasts were transdifferentiated to myoblasts for 72 hours.

After 72 h, total protein used for QDB assays was extracted using RIPA buffer (Thermo Scientific) plus protease and phosphatase inhibitors (Roche Applied Science). Samples were quantified by BCA protein assay kit (Pierce) using bovine serum albumin as a standard. Samples were prepared at a concentration of 1 µg/well along with 4x loading buffer (0.8 ml 3 M Tris-HCl pH 6.8, 4 ml glycerol, 0.8 g SDS, 0.04 g bromophenol blue, 4 ml β-mercaptoethanol, ddH₂O up to 10 ml). Samples were denatured by boiling at 100°C for 5 min and loaded onto QDB plates (Quanticision Diagnostics Inc). Each sample was loaded in quadruplicate on two different plates (5 µl/well), one to detect MBNL1 and the other to detect GAPDH, used as endogenous control, following the protocol described in Moreno-Cervera *et* al., 2022. After this, they were left to dry and were incubated with transfer buffer (14.4 g glycine, 3 g Tris base, 700 ml ddH₂O, 200 ml methanol, ddH₂O up to 1 I) under stirring for 1 min. This was followed by three washes with 1x TBS-T (100 ml 10x TBS, 5 ml Tween 20, ddH₂O up to 1 l) and incubated for 1 hour with blocking buffer (5 g skim milk powder, 1x TBS-T up to 100 ml). Plates were incubated at 4°C overnight with anti-MBNL1 (1:1000, ab77017, Abcam) and anti-GAPDH (1:500, clone G-9, Santa Cruz) primary antibodies. All primary antibodies were detected using HRP (horseradish peroxidase)-conjugated anti-mouse IgG secondary antibody at a concentration of 1:5000 (Sigma-Aldrich). Immunoreactivity was detected using Pierce ECL Western Blotting Substrate reagent (Thermo Scientific) and chemiluminescence levels were determined with the Inifinite M200 Pro plate reader (Tecan).

### Expression of candidate miRNAs in the relevant tissues

The expression levels of miR-100-5p, miR-20a-5p, miR-181c-5p miR-222-3p, and miR-92a-3p in transcripts per million (TPM), from the different tissues of interest (trachea, thyroid, testicles, stomach, spleen, different types of muscle cells, skeletal muscle, liver, kidney, heart, bladder, esophagus, diaphragm, colon, cervix, brain, and blood) were obtained by means of using the database, which is created by means of data from the project. Fantom hosts a large number of samples of more than 100 different types of tissue, therefore those which may be of interest for the disease have been filtered.

| Name | TC50 | EC50 | Emax | TIndex |
|---|---|---|---|---|
| **AntagomiR-23b** | 0.806 | 0.0278 | 2.596 | **75.254** |
| **AntagomiR-218** | 0.848 | 0.0224 | 1.870 | **70.810** |
| **AntagomiR-20a** | 0.340 | 0.0100 | 1.458 | **49.572** |
| **AntagomiR-92a** | 1.201 | 0.4170 | 2.434 | **7.010** |
| **AntagomiR-100** | 1.524 | 0.0180 | 1.552 | **131.622** |
| **AntagomiR-181c** | 3.849 | 0.4675 | 1.340 | **11.034** |
| **AntagomiR-222** | 0.579 | 0.9777 | 1.979 | **1.172** |

**Therapeutic** index **of the different oligonucleotides used in DM1 cells.** TC50, EC50, Emax, and therapeutic index parameters obtained for each of the antagomiRs tested in DM1 cells.

### Expression of candidate miRNAs in disease model cells

The relative expression levels of microRNAs miR-23b, miR-218, miR-92a, mirR-100, miR-181c, miR-222, miR-20a, miR-17, and miR-let7a were measured by means of RT-qPCR both in control cells and in DM1 model cells after 4 days of differentiation. Levels of miR-1 were also measured as a positive control, since it is described (Rau *et al.,* 2011) that miR-1 is reduced in DM1 cells. All the levels were obtained using U1, U6, and miR-103b as normalizers.

| | *Screening mkroRNAS* | | *No. 3'UTR binding prediction programs* | | | *qPCR Validation* | | *Expression in tissues* | *Overexpression in DM1* |
|---|---|---|---|---|---|---|---|---|---|
| *miRs* | ↓MB NL1 | ↓MBNL2 | MBNL1 | MBNL2 | TarBase v7.0 | ↓MBNL 1 | ↓MBNL 2 | muscle | myotubes |
| *hsa-miR-20a* | Yes | Yes | 5 | 4 | | | | Yes | Yes |
| *hsa-miR-181c* | | | 8 | | + | Yes | | Yes | Yes |
| *hsa-miR-222* | Yes | Yes | 4 | 4 | + | | | Yes | Yes |
| *hisa-miR-100* | Yes | Yes | 6 | | | | | Yes | Yes |
| *hsa-miR-92a* | Yes | Yes | 3 | 3 | + | | | Yes | Yes |

**Table 3. Compilation of data obtained on the different miRNAs under study.** The different parameters of interest analyzed in the study are shown: confirmation of MBNL1 and 2 reduction in the overexpression screening of the miRNAs under study; number of programs predicting the binding of miRNAs to MBNL1 and 2 and confirmation by TarBase v7.0; validation of MBNL1 and 2 reduction by qPCR when the miRNAs of interest are overexpressed; confirmation of miRNA expression in tissues of interest for DM1 (muscle); and confirmation of miRNA overexpression in DM1 myotubes model of the disease.

As can be seen in Table 2, antagomiR-100 showed the best Tindex values with respect to the rest of the antagomiRs, which shows that this antagomiR presents the best balance between toxicity, Emax, and EC50. However, it should also be noted that antagomiR-20b has good EC50 and Emax values, antagomiR-181c has a good Emax value, and antagomiR-92a has good TC50 and Emax values comparable to antagomiR-23b. Note that low EC50 values indicate higher antagonistic potency of the miRNA in question, while higher TC50 indicates lower toxicity on the cell model of the disease.

Furthermore, protein expression levels were studied in the cell model of the disease (Figure 3) finding, firstly, that DM1 muscle cells express significantly less MBNL1 protein than the control. Taking as a reference the expression detected in DM1 cells, it was found that in all cases an increase in total protein levels occurs, although some antagomiRs are active at lower concentrations than others and the levels of activation may be different. For example, for antagomiR-20a or -100, at 10 nM (the lowest concentration tested), recovery in MBNL1 expression reaches normal levels, whereas other antagomiRs are able to double expression in DM1 cells (antagomiR-92a and -222). This indicates that some antagomiRs are more effective than others, such as antagomiR-181c, the activation of MBNL1 expression of which is discrete, and requires higher concentration to achieve levels similar to those of the rest of the molecules.

Figure 4 shows the evaluation of the activity and toxicity of the different antagomiRs in DM1 cells after transfection with same at the different concentrations indicated. The different graphs visually show the Tindex metric given for those antagomiRs for which the EC and TC curves are farther apart; these are the ones that that show the greatest difference between the concentrations at which they are active and those at which they show toxic effects (therapeutic window). For example, antagomiR-100 shows activity at low concentrations and rapidly reaches the maximum possible activity, while only at concentrations almost two orders of magnitude higher do they show a reduction by half in the viability of DM1 muscle cells. This behavior is similar to that of antagomiR-23b and -218, which are shown in comparison. In contrast, antagomiR-222 can increase MBNL1 expression markedly, but does so at concentrations at which the toxicity curve is already above 50% dead cells. The rest of the antagomiRs show an intermediate behavior. AntagomiR-20a also shows an interesting behavior since at the lowest concentration tested it already reaches the maximum activity in terms of MBNL1 activation, with its TC50 being at least one order of magnitude higher. In antagomiR-181c, the difference between activity and toxicity is low. Taking both curves together, it is observed that antagomiR-100 presents a similar pattern to the previously patented antagomiRs, -23b and -218, since its activity levels are very high from the second dose tested, but its toxicity level exceeds the TC50 only at high doses, i.e., it presents a wider therapeutic window and therefore is more suitable for pharmacological development.

Next, the expression of the microRNAs of interest was determined in different human tissues (Figure 5). As can be seen, microRNA miR-100 is expressed almost exclusively in skeletal muscle and its satellite cells, with only some expression in fibroblasts, which is of particular interest because skeletal muscle is a tissue that is greatly affected in DM1, so that the antagonistic effect of antimiR-100 is expected to be specific, avoiding possible undesirable consequences due to repression of this miRNA in tissues that are not altered in the disease. For example, since antimiRs accumulate in the liver and kidney, the fact that the antimiR-100 target is not expressed in these organs will prevent reaching a lack of function situation of this miRNA in these organs, which is desirable from a therapeutic point of view. On the other hand, the expression of miR-100 in satellite cells is also of great interest because these are the muscle stem cells from which muscle regeneration can be promoted. In fact, it has been demonstrated (Song KY, *et al.* MBNL1 reverses the proliferation defect of skeletal muscle satellite cells in myotonic dystrophy type 1 by inhibiting autophagy via the mTOR pathway. Cell Death Dis. 2020 Jul 18;11(7):545. doi: 10.1038/s41419-020-02756-8. PMID: 32683410; PMCID: PMC7368861) that an increase of MBNL1 in these cells reverses the proliferative defects that these cells have in DM1, so acting at the satellite cell level through a satellite cell-specific target is anticipated to be potentially therapeutic *in vivo.* Finally, the detection of miR-100 in mature skeletal muscle confirms the relevance of the observations made *in vitro* with cell models of the disease, which may differ markedly with the *in vivo* expression pattern in adult humans.

Finally, an assay was performed to quantify possible differences in expression of the different target miRNAs in cell models of the disease with respect to their healthy controls. The results in Figure 6 show that most of the selected miRNAs are detected overexpressed in an *in vitro* model of the disease, with this characteristic being desirable in a therapeutic target because it reduces the risk of harmful effects due to lack of target function. That is, the preferable therapeutic target is not only to reduce the expression of an miRNA below its normal values, but to bring it to levels similar to those of healthy controls, thus preserving its natural endogenous functions. As a control, note that miR-218, previously described as overexpressed in DM1 (see Cerro-Herreros E et al. Preclinical characterization of antagomiR-218 as a potential treatment for myotonic dystrophy. Mol Ther Nucleic Acids. 2021 Jul 29;26:174-191. doi: 10.1016/j.omtn.2021.07.017. PMID: 34513303; PMCID: PMC8413838), is detected as being increased in this cell model, as well as miR-23b, while miR-17 and others do not show any significant change in expression and serve as a control that this is not a general effect on all miRNAs analyzed.

## Claims

1. An antagonist of human microRNA miR-100 capable of increasing the intracellular levels of MBNL1 and/or MBNL2 proteins, for use thereof in the treatment of myotonic dystrophy type 1.

2. The antagonist of human microRNA miR-100 for use according to claim 1, wherein the intracellular levels of MBNL1 and/or MBNL2 proteins are increased in a muscle cell, preferably in a muscle cell having a muscular dystrophy phenotype, preferably myotonic dystrophy type 1.

3. The antagonist of human microRNA miR-100 for use according to any of claims 1 or 2, wherein said antagonist is a molecule of an oligonucleotide nature and/or an oligonucleotide analog.

4. The antagonist of human microRNA miR-100 for use according to any of claims 1 to 3, wherein said antagonist is an antimiR, a blockmiR, or a microRNA sponge.

5. The antagonist of human microRNA miR-100 for use according to any of claims 1 to 4, wherein said antagonist comprises a region which is at least 85% identical to the complementary sequence of SEQ ID NO: 1.

6. The antagonist of human microRNA miR-100 for use according to any of claims 1 to 5, wherein said antagonist comprises a region of at least 7 contiguous nucleotide or nucleotide analog units which is 100% identical to the sequence complementary to the seed region of human microRNA miR-100 as defined in SEQ ID NO: 2.

7. The antagonist of human microRNA miR-100 for use according to any of claims 1 to 6, wherein said antagonist comprises a first region of at least 7 contiguous nucleotide or nucleotide analog units which is 100% identical to the sequence complementary to the seed region of human microRNA miR-100 as defined in SEQ ID NO: 2, and wherein said antagonist further comprises a second region of at least 7 contiguous nucleotide or nucleotide analog units adjacent to the first region, wherein said second region is at least 90% identical to the sequence complementary to any region present in SEQ ID NO: 1.

8. The antagonist of human microRNA miR-100 for use according to any of claims 1 to 7, wherein said antagonist comprises a nucleotide or nucleotide analog region which is at least 90% identical to SEQ ID NO: 3.

9. The antagonist of human microRNA miR-100 for use according to any of claims 1 to 8, wherein:
i) said antagonist comprises a region which is at least 95% identical to SEQ ID NO: 3,
ii) at least one of the nucleotide or nucleotide analog units making up said antagonist is a nucleotide analog having one or more chemical modifications in the ribose moiety, in the phosphate bond, or in both, and
iii) optionally, said antagonist has at the 5' end and/or at the 3' end one or more additional molecules, preferably cholesterol.

10. The antagonist of human microRNA miR-100 for use according to any of claims 1 to 9, wherein said antagonist comprises a nucleotide or nucleotide analog region which is at least 95% identical to any of the sequences SEQ ID NO: 8, 13, or 26.

11. The antagonist of human microRNA miR-100 for use according to any of claims 9 or 10, wherein said antagonist consists of SEQ ID NO: 8, SEQ ID NO: 13, or SEQ ID NO: 26.

12. A composition comprising at least one antagonist of human microRNA miR-100 for use thereof in the treatment of myotonic dystrophy type 1.

13. The composition for use according to claim 12, wherein said composition is a pharmaceutical composition further comprising a pharmaceutically acceptable vehicle and/or one or more pharmaceutically acceptable excipients.
